# EUROPEAN PATENT APPLICATION

(11) **EP 4 714 387 A1**
(43) Date of publication of application: **25.03.2026**
(21) Application number: 25202994.7
(22) Date of filing: 18.09.2025
(51) Int. Cl.: A61B 34/20

(54) **INTERACTIVE TECHNIQUES FOR CO-REGISTRATION OF EXTENDED REALITY AND SURGICAL NAVIGATION SYSTEMS**

(30) Priority: 19.09.2024 US 202463696475 P
(71) Applicant: Stryker Corporation, Portage, MI 49002 (US)
(72) Inventor: Herrmann, Dr. Florian, 77963 Schwanau (DE); Harrow, Matthew E., Mooresville, 28117 (US)
(74) Representative: Schott, Jakob Valentin

(57) **Abstract**

Techniques for co-registration of extended reality and surgical navigation systems involve detection of a spatial relationship between a virtual registration object and a physical registration object (e.g., tool). The physical registration object is observed on, or through, the display of the head-mounted device (HMD). In one implementation, biomechanical control input (e.g., hand/finger or eye tracking) from the HMD user is sensed to command acquisition of the virtual registration object relative to the physical registration object. In another implementation, the virtual registration object is presented on the HMD display for alignment with the physical registration object. In some instances, a virtual guide object is presented on the HMD display to dynamically guide the user to align the physical and virtual registration objects.

## Description

### CROSS-REFERENCE TO RELATED APPLICATIONS

The subject application claims priority to and all benefits of United States Provisional Patent App. No. 63/696,475, filed September 19, 2024, the entire contents of which are hereby incorporated by reference.

### BACKGROUND

The integration of extended reality (XR) technologies has significantly transformed various industries, such as the fields of medicine and surgery. For example, surgical theatres have witnessed a remarkable shift in how XR technologies are used to enhance patient care, surgical outcomes, and the overall surgical experience. XR encompasses virtual reality (VR), augmented reality (AR), and mixed reality (MR), each of which has its unique applications and advantages within the surgical setting. Extended reality in surgical theatres brings numerous benefits to both surgeons and patients. Extended reality technologies offer surgeons the opportunity to practice, plan, and refine surgical procedures before entering the operating room. Virtual reality allows surgeons to immerse themselves in a 3D reconstruction of the patient's anatomy, enabling them to explore the intricacies of the body and anticipate potential challenges. This preoperative practice not only enhances surgical precision but also reduces the likelihood of complications during the actual procedure.

For real-time surgical navigation and guidance, intraoperative XR systems provide surgeons with real-time guidance and visualization during surgery. By overlaying important surgical information, such as 3D anatomical models, vital signs, or instrument tracking, onto the surgeon's field of view, XR helps ensure precision and efficiency during surgery. Surgeons can use this guidance to make informed decisions and navigate complex structures with greater ease.

To enable the use of XR in real-time surgical navigation, co-registration of the XR and navigation worlds is required. However, conventional co-registration techniques have several shortcomings. Prior techniques often rely on specialized hardware, such as calibration devices with markers detectable by the navigation system placed in a fixed array relative to markers detectable by the camera of the XR headset. Such calibration devices add cost to the system as they introduce extra hardware and require additional steps and time to complete co-registration. Similar types of specialized hardware include trackers that are attached to the XR headset and that are detectable by the navigation system. However, such trackers similarly introduce cost and complexity. For example, there are numerous manufacturers of XR headsets, and the tracker may not be configured to easily attach to, or work with, the specific make/model of XR headset that the surgeon desires to use. Moreover, the addition of a specialized tracker on the XR headset can introduce extra mass and size to the headset, thereby causing potential discomfort for the surgeon and collisions with other objects in the operation room. In turn, providing a navigation tracker for the XR headset may not be an optimal means for co-registering the XR and navigation systems. Accordingly, conventional co-registration techniques often fail to provide a cost-effective solution that does not require the inclusion of specialized components.

Moreover, prior co-registration techniques also fail to take full advantage of specialized XR features for detecting bio-mechanical input, such as gesture or gaze of the headset user. For example, many prior techniques require the user to manually manipulate a tracked pointer to touch on numerous pre-determined physical or virtual points with the pointer tip. The user must precisely probe such points with the pointer or else the system will register inaccuracy errors. Having to wear the XR headset during conventional point acquisition can be challenging because the user must manually acquire points with a physical pointer while observing the pointer through an XR display, which may be a video stream of the real-world environment. Therefore, such processes can be cumbersome on the user. Moreover, prior techniques fail to provide sufficient interactive guidance to aid the user in performing co-registration.

There is a need to provide techniques for co-registration of XR and surgical navigation systems that are intuitive to the headset wearer, efficient and accurate, take advantage of bio-mechanical control inputs, and do not require the introduction of specialized components.

### SUMMARY

This Summary introduces a selection of concepts in a simplified form that are further described below in the Detailed Description below. This Summary is not intended to limit the scope of the claimed subject matter nor identify key features or essential features of the claimed subject matter.

According to a first aspect, a surgical system is provided, comprising: a navigation system comprising a localizer including a localizer coordinate system, wherein the localizer is configured to track a physical registration object such that a pose of the physical registration object is known in the localizer coordinate system; a head-mounted device (HMD) comprising an HMD coordinate system, a display positionable in front of the eyes of a user of the HMD, and a sensing system configured to sense a biomechanical control input from the user, wherein the HMD is configured to: enable the physical registration object to be observed on, or through, the display; and sense, with the sensing system, the biomechanical control input from the user to command acquisition of a virtual registration object relative to the physical registration object; and one or more controllers coupled to the navigation system and/or the HMD and being configured to utilize the virtual registration object to co-register the HMD coordinate system and the localizer coordinate system.

According to a second aspect, a surgical system is provided, comprising: a navigation system comprising a localizer including a localizer coordinate system, wherein the localizer is configured to track a physical registration object such that a pose of the physical registration object is known in the localizer coordinate system; a head-mounted device (HMD) comprising an HMD coordinate system, a display positionable in front of the eyes of a user of the HMD, and wherein the HMD is configured to: enable the physical registration object to be observed on, or through, the display; detect the physical registration object and obtain a pose of the physical registration object in HMD coordinate system; present a virtual registration object on the display, wherein a pose of the virtual registration object is known in the HMD coordinate system; and one or more controllers coupled to the navigation system and/or the HMD and being configured to: detect alignment between the pose of the physical registration object and the pose of the virtual registration object; and determine a relationship between the HMD coordinate system and the localizer coordinate system in response to detection of alignment between the pose of the physical registration object and the pose of the virtual registration object.

According to a third aspect, a surgical system is provided, comprising: a navigation system comprising a localizer including a localizer coordinate system, wherein the localizer is configured to track a physical registration object such that a pose of the physical registration object is known in the localizer coordinate system; a head-mounted device (HMD) comprising an HMD coordinate system, a display positionable in front of the eyes of a user of the HMD, and wherein the HMD is configured to: enable the physical registration object to be observed on, or through, the display; present a virtual registration object on the display, wherein a pose of the virtual registration object is known in the HMD coordinate system and wherein the virtual registration object is virtually anchored in a real-world coordinate system; and one or more controllers coupled to the navigation system and/or the HMD and being configured to co-register the HMD coordinate system and the localizer coordinate system in response to detection of alignment between the pose of the physical registration object and the pose of the virtual registration object.

According to a fourth aspect, a surgical system is provided, comprising: a navigation system comprising a localizer including a localizer coordinate system, wherein the localizer is configured to track a physical registration object such that a pose of the physical registration object is known in the localizer coordinate system; a head-mounted device (HMD) comprising an HMD coordinate system, a display positionable in front of the eyes of a user of the HMD, and a sensing system configured to sense a gaze of the user, wherein the HMD is configured to: enable the physical registration object to be observed on, or through, the display; and sense, with the sensing system, the gaze of the user to command movement of a virtual registration object into alignment with the physical registration object; and one or more controllers coupled to the navigation system and/or the HMD and being configured to co-register the HMD coordinate system and the localizer coordinate system in response to detection of alignment between the virtual registration object and the physical registration object.

According to a fifth aspect, a surgical system is provided, comprising: a navigation system comprising a localizer including a localizer coordinate system, wherein the localizer is configured to track a surgical tool such that a pose of the surgical tool is known in the localizer coordinate system, wherein the surgical tool comprises a tool tip; a head-mounted device (HMD) comprising an HMD coordinate system, a display positionable in front of the eyes of a user of the HMD, and wherein the HMD is configured to: enable the tool tip of the surgical tool to be observed on, or through, the display; present a virtual registration point on the display, wherein a pose of the virtual registration point is known in the HMD coordinate system; and one or more controllers coupled to the navigation system and/or the HMD and being configured to co-register the HMD coordinate system and the localizer coordinate system in response to detection of alignment between the tool tip and the of the virtual point.

According to a sixth aspect, a surgical system is provided, comprising: a navigation system comprising a localizer including a localizer coordinate system, wherein the localizer is configured to track a physical registration object such that a pose of the physical registration object is known in the localizer coordinate system; a head-mounted device (HMD) comprising an HMD coordinate system, a display positionable in front of the eyes of a user of the HMD, and wherein the HMD is configured to: enable the physical registration object to be observed on, or through, the display; present a virtual registration object on the display, wherein a pose of the virtual registration object is known in the HMD coordinate system; and present a virtual guide object on the display, wherein the virtual guide object is configured to dynamically guide the user to align a pose of the physical registration object and the pose of the virtual registration object; and one or more controllers coupled to the navigation system and/or the HMD and being configured to co-register the HMD coordinate system and the localizer coordinate system in response to detection of alignment between the pose of the physical registration object and the pose of the virtual registration object.

Also provided are: a method of operating the surgical system of any aspect above; a method of operating the HMD of any aspect above; a non-transitory computer-readable medium (or computer program product) comprising instructions, which when executed by one or more processors, operate the surgical system of any aspect above; a non-transitory computer-readable medium (or computer program product) comprising instructions, which when executed by one or more processors, operate the HMD of any aspect above; and a computer-implemented method of co-registering the HMD of any aspect above to the navigation system of any aspect above.

Any of the above aspects may be combined, in whole or in part.

Any of the above aspects may be combined with any of the following implementations. Any of the following implementations may be utilized in part, or in whole, with any of the above aspects. The implementations are:

The biomechanical control input can include detecting the gaze of the user focused on the physical registration object and/or detecting a hand or finger of the user physically touching the physical registration object. The virtual registration object can be acquired in response to sensing, with the sensing system, the biomechanical control input for a threshold amount of time.

The physical registration object can be any surgical object. The surgical object can be the patient anatomy or any surgical object that is separated and disconnected from a patient anatomy. The physical registration object can be a surgical tool that is detectable by the localizer or a feature of the tool that is known to the localizer. The physical registration object can be a tracker that is detectable by the localizer. The physical registration object can have a reference datum. A pose of the reference datum can be known in the localizer coordinate system. The HMD can sense, with the sensing system, the biomechanical control input from the user to command acquisition of the virtual registration object that corresponds to the reference datum.

The HMD can detect the physical registration object, for example, using biomechanical control input (gaze/gesture) and by detecting the physical registration object using a camera of the HMD. The HMD can obtain a pose of the physical registration object in HMD coordinate system. The HMD can obtain the pose of the physical registration object in HMD coordinate system to perform a coarse registration or initial registration. The one or more controllers can detect alignment between the pose of the physical registration object and the pose of the virtual registration object to perform a fine registration, a re-registration, and/or verification of an initial registration. For any implementation, the one or more controllers can include, but are not limited to: the HMD controller 210, the navigation controller 26, and/or any controller of an intermediary device.

The virtual registration object can be combined with a real-world view presented on or through the HMD display. The virtual registration object can be of any geometry, including a point, a line, a plane, a volume, or a point cloud. The virtual registration object can be shaped to mimic an entirety, or a portion of the physical registration object. The virtual registration object can be acquired relative to the surgical object, such as a tool, tracker, anatomy, camera unit, etc. The virtual registration object can be acquired relative to a feature or sub-component of the surgical object. The virtual registration object can be acquired relative to a real-world view of the physical registration object. The virtual registration object can be a 3D virtual representation of the anatomy.

The virtual registration object can be virtually anchored in a real-world coordinate system, and the physical registration object can be brought into alignment with the virtual registration object. The virtual registration object can be virtually anchored in the HMD coordinate system, and the physical registration object can be brought into alignment with the virtual registration object, or vice versa. The virtual registration object can be virtually moved in the HMD coordinate system to bring the virtual registration object into alignment with the physical registration object.

Prior to co-registration between the HMD coordinate system and the localizer coordinate system, a pose the HMD may be unknown in the localizer coordinate system. After co-registration between the HMD coordinate system and the localizer coordinate system, the pose the HMD may be known in the localizer coordinate system. The HMD can be trackerless such that the HMD is not directly trackable by the navigation system.

The HMD can include a camera configured to capture a video of a real-world view and the HMD is configured to present the video of the real-world view on the display. The HMD display may be a transparent or semi-transparent display such that the real-world view is visible by the eyes of the user based on light passing directly through the display.

The HMD can present a virtual guide object on the display. The virtual guide object is configured to dynamically guide the user to align a pose of the physical registration object and the pose of the virtual registration object. The virtual guide object can be any one or more of: a virtual arrow; a virtual path; a virtual animation; a virtual representation of a 3D coordinate system; a virtual compass; and a virtual reticle. The virtual guide object can include a visual feature that is configured to dynamically change responsive to changes in a spatial relationship between the pose of the physical registration object and the pose of the virtual registration object. The visual feature of the virtual guide object that dynamically changes includes one or more of: a pose; a color; an opacity; a shape; a size; a length; a direction; and/or a magnitude of the virtual guide object. The virtual guide object can alternatively or additionally include text that is displayed on the HMD display and that dynamically changes responsive to changes in the spatial relationship between the pose of the physical registration object and the pose of the virtual registration object. The text can include: alignment instructions; a measurement of displacement between the physical registration object and the virtual registration object; a measurement of direction between the physical registration object and the virtual registration object; an indication of misalignment; and/or a confirmation of alignment. The virtual guide object can be presented as being part of the virtual registration object and/or can augment the virtual registration object. The virtual guide object can be configured to guide the user with respect to multiple degrees of freedom, up to six degrees of freedom. Any of the implementations above can be combined in part, or in whole, and any of the above aspects.

### BRIEF DESCRIPTION OF THE DRAWINGS

Advantages of the present invention will be readily appreciated as the same becomes better understood by reference to the following detailed description when considered in connection with the accompanying drawings wherein:
FIG. 1 is a perspective view of a surgical system, according to one implementation.
FIG. 2 is a schematic view of an example control system that can be used with the surgical system.
FIG. 3 is an illustration of various coordinate systems and transforms that can be established relative to the various components of the surgical system, according to one implementation.
FIG. 4 is a method of techniques for co-registering a head-mounted device HMD and a navigation system, according to one implementation.
FIGS. 5A and 5B are sample first-person views through the HMD display whereby an HMD user is performing the co-registration technique by acquiring a point on a pointer tool using eye-tracking, according to one implementation.
FIGS. 6A and 6B are sample first-person views through the HMD display whereby an HMD user is performing the co-registration technique by acquiring a point on a pointer tool using hand-tracking, according to one implementation.
FIG. 7 is sample first-person view through the HMD display whereby an HMD user is performing the co-registration technique by acquiring a point on an anatomy tracker using hand and/or eye tracking, according to one implementation.
FIG. 8 is sample first-person view through the HMD display whereby an HMD user is performing the co-registration technique by acquiring a point on one or more robot trackers using hand and/or eye tracking, according to one implementation.
FIG. 9 is sample first-person view through the HMD display whereby an HMD user is performing the co-registration technique by acquiring points on a bone surface using hand and/or eye tracking, according to one implementation.
FIGS. 10A and 10B are sample first-person views through the HMD display whereby an HMD user is performing the co-registration technique by acquiring a point on a camera unit using hand and/or eye tracking, according to one implementation.
FIGS. 11A and 11B are sample first-person views through the HMD display whereby an HMD user is performing the co-registration technique by aligning a tool tip and a virtual point, according to one implementation.
FIGS. 12A and 12B are sample first-person views through the HMD display whereby an HMD user is performing the co-registration technique by aligning a saw blade and a virtual plane, according to one implementation.
FIGS. 13A and 13B are sample first-person views through the HMD display whereby an HMD user is performing the co-registration technique by aligning a virtual outline to a camera unit, according to one implementation.

### DETAILED DESCRIPTION

### I. Example System Overview

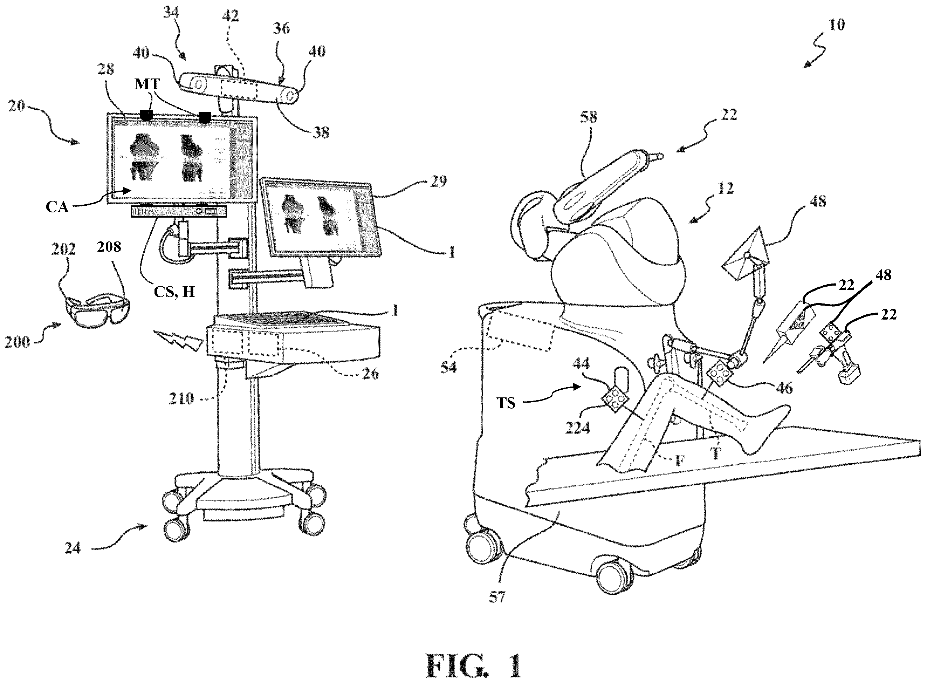

Referring to FIG. 1, a system 10 is provided. The system can be a surgical system 10 adapted for treating a patient. The surgical system 10 is shown in a surgical setting such as an operating room of a medical facility. The surgical system 10 may be used to perform any intraoperative surgical procedure on a patient. Example surgical procedures include, but are not limited to: partial knee arthroplasty, total knee arthroplasty, total hip arthroplasty, shoulder arthroplasty, spinal procedures, ankle procedures, endoscopic procedures, cranial procedures, lesion removal procedures, arthroscopic procedures, arthroscopic resection procedures, soft tissue or ligament repair procedures, neurological procedures, ENT procedures, minimally invasive MIS procedures, or the like. In the example shown in FIG. 1, the patient is undergoing a knee procedure. In addition, the following implementations describe the use of the surgical system 10 in performing a procedure in which material is removed from a femur F and/or a tibia T of a patient. However, the surgical system 10 may be used to perform any suitable procedure in which material is removed from any suitable portion of a patient's anatomy, material is added to any suitable portion of the patient's anatomy (e.g., an implant, graft, etc.), and/or in which any other control of and/or visualization of a surgical tool is desired.

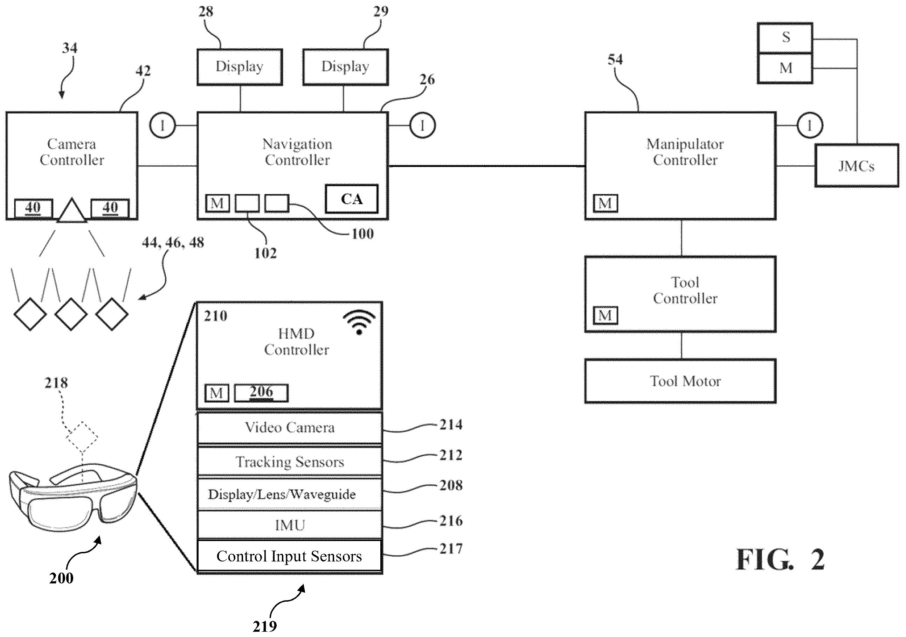

In the implementation shown, the surgical system 10 includes a manipulator 12 (e.g., surgical robot) and a navigation system 20. The navigation system 20 is set up to track movement of various objects in the operating room. Such objects include, for example, one or more surgical tools 22 and a target site TS of the patient (e.g., a femur F and a tibia T). The navigation system 20 tracks these objects for purposes of displaying their relative positions and orientations to the surgeon on a clinical application (CA) and, in some cases, for purposes of controlling or constraining movement of the surgical tool 22 relative to virtual cutting boundaries (VB) associated with the target site TS. An example control scheme for the surgical system 10 is shown in FIG. 2.

In the implementation shown, the surgical tool 22 is attached to the manipulator 12. Such an arrangement is shown in U.S. Patent No. 9,119,655, entitled, "Surgical Manipulator Capable of Controlling a Surgical Instrument in Multiple Modes," the disclosure of which is hereby incorporated by reference. In one example, the manipulator 12 has a base 57, a plurality of links 58 extending from the base 57, and a plurality of joints (not numbered) for moving the surgical tool 22 with respect to the base 57. The links 58 and joints form a robotic arm. Some or all of the joints may be passive joints or active joints. The manipulator 12 may have a serial arm or parallel arm configuration. The manipulator 12 can be floor mounted, ceiling mounted, gantry mounted, table mounted, or patient mounted. More than one manipulator 12 can be utilized.

While the surgical system 10 is illustrated in FIG. 1 as including the surgical tool 22 attached to the manipulator 12, it should be recognized that the surgical system 10 may additionally or alternatively include one or more manually operated or hand-held surgical tools 22. For example, the surgical tool 22 may include a hand-held motorized saw/drill/bur/driver, a hand-held probe/pointer/digitizer, or other suitable tool that may be held and manually operated by a surgeon. Any implementations described with reference to the use of the manipulator 12 may also apply to the use of a hand-held tool 22 with appropriate modifications.

The navigation system 20 includes one or more computer cart assemblies 24 that houses one or more navigation controllers 26. A navigation interface is in operative communication with the navigation controller 26. The navigation interface includes one or more displays 28, 29 adjustably mounted to the computer cart assembly 24 or mounted to separate carts as shown. Input devices I such as a keyboard and mouse can be used to input information into the navigation controller 26 or otherwise select/control certain aspects of the navigation controller 26. Other input devices I are contemplated including a touch screen, a microphone for voice-activation input, an optical sensor for gesture input, and the like.

The clinical application CA can be displayed on one or more displays 28, 29 of the navigation system 20. The clinical application CA assists a surgeon or staff in performing the surgical procedure. The clinical application CA can have a plurality of different screens related to the surgical procedure. Such screens can include a pre-operative planning screen, an operating room setup screen, an anatomical registration screen, an intra-operative planning screen, an anatomical preparation screen, or a post-operative evaluation screen, and the like. The clinical application CA can present a navigation guidance region that displays one or more of the surgical objects tracked by a localizer 34 of the navigation system 20.

The localizer 34 communicates with the navigation controller 26. In the implementation shown, the localizer 34 is an optical localizer and includes a camera unit 36. The camera unit 36 has a housing 38 comprising an outer casing that houses one or more optical sensors 40. The optical sensors 40 can detect light signals, such as infrared (IR) signals and/or visible light signals. Camera unit 36 can be mounted on an adjustable arm to position the optical sensors 40 with a field-of-view of the below discussed trackers that, ideally, is free from obstructions. The camera unit 36 includes a camera controller 42 in communication with the optical sensors 40 to receive signals from the optical sensors 40. The camera controller 42 communicates with the navigation controller 26 through either a wired or wireless connection (not shown). In other implementations, the optical sensors 40 communicate directly with the navigation controller 26. Position and orientation signals and/or data are transmitted to the navigation controller 26 for purposes of tracking objects. The computer cart assembly 24, display 28, and camera unit 36 may be like those described in U.S. Patent No. 7,725,162 to Malackowski, et al. issued on May 25, 2010, entitled "Surgery System," the disclosure of which is hereby incorporated by reference. The navigation controller 26 can be a personal computer or laptop computer. Navigation controller 26 includes the displays 28, 29, central processing unit (CPU) and/or other processors, memory (not shown), and storage (not shown). The navigation controller 26 is loaded with software that converts the signals received from the camera unit 36 into data representative of the position and orientation of the objects being tracked. The navigation controller 26 includes a navigation processor. It should be understood that the navigation processor could include one or more processors to control operation of the navigation controller 26. The processors can be any type of microprocessor or multi-processor system. The term processor is not intended to limit the scope of any implementation to a single processor.

Navigation system 20 is operable with a plurality of tracking devices 44, 46, 48, also referred to herein as trackers. In the illustrated implementation, one or more trackers 44, 46 can be patient/anatomy/target site trackers, e.g., tracker 44 coupled to the femur F and another tracker 46 can be firmly affixed to the tibia T. Trackers 44, 46 are firmly affixed to sections of bone in an implementation. For example, trackers 44, 46 may be attached to the femur F and tibia T in the manner shown in U.S. Patent No. 7,725,162 to Malackowski, et al. issued on May 25, 2010, entitled "Surgery System," the disclosure of which is hereby incorporated by reference. Trackers 44, 46 may also be mounted like those shown in U.S. Patent Application No. 14/156,856, filed on January 16, 2014, entitled, "Navigation Systems and Methods for Indicating and Reducing Line-of-Sight Errors," hereby incorporated by reference herein. The trackers 44, 46 may be mounted to other tissue types or parts of the anatomy. A tool tracker 48 can be coupled to the manipulator 12 or the tool 22 at any suitable location. The tool tracker 48 can be integrated into the surgical tool 22 during manufacture or may be separately mounted to the surgical tool 22 (or to an end effector attached to the manipulator 12 of which the surgical tool 22 forms a part) in preparation for surgical procedures. The working end of the surgical tool 22, which is being tracked by virtue of the tool tracker 48, may be referred to herein as an energy applicator, and may be a rotating bur, saw, router, reamer, impactor, electrical ablation device, cut guide, tool holder, probe, or the like.

In one implementation, optical sensors 40 of the localizer 34 receive light signals from the trackers 44, 46, 48. In one example, the trackers 44, 46, 48 are passive trackers. In this implementation, each tracker 44, 46, 48 has at least three passive tracking elements or markers (e.g., reflectors) for transmitting light signals (e.g., reflecting ambient light or light emitted from the camera unit 36) to the optical sensors 40. In other implementations, active tracking markers can be employed. The active markers can be, for example, light emitting diodes transmitting light, such as infrared light. Active and passive arrangements are possible. The camera unit 36 receives optical signals from the trackers 44, 46, 48 and outputs to the navigation controller 26 signals relating to the position of the tracking markers of the trackers 44, 46, 48 relative to the localizer 34. Based on the received optical signals, navigation controller 26 generates data indicating the relative positions and orientations of the trackers 44, 46, 48 relative to the localizer 34. These relative positions can be displayed on the clinical application CA as graphical representations for surgical guidance.

In another implementation, the navigation system 20 and/or the localizer 34 are radio frequency (RF) based. For example, the navigation system 20 may comprise an RF transceiver coupled to the navigation controller 26. Here, the trackers 44, 46, 48 may comprise RF emitters or transponders, which may be passive or may be actively energized. The RF transceiver transmits an RF tracking signal, and the RF emitters respond with RF signals such that tracked states are communicated to (or interpreted by) the navigation controller 26. The RF signals may be of any suitable frequency. The RF transceiver may be positioned at any suitable location to track the objects using RF signals effectively. Furthermore, examples of RF-based navigation systems may have structural configurations that are different than the navigation system 20 illustrated throughout the drawings.

In other examples, the navigation system 20 and/or localizer 34 are electromagnetically (EM) based. For example, the navigation system 20 may comprise an EM transceiver coupled to the navigation controller 26. Here, the trackers 44, 46, 48 may comprise EM components attached thereto (e.g., various types of magnetic trackers, electromagnetic trackers, inductive trackers, and the like), which may be passive or may be actively energized. The EM transceiver generates an EM field, and the EM components respond with EM signals such that tracked states are communicated to (or interpreted by) the navigation controller 26. The navigation controller 26 may analyze the received EM signals to associate relative states thereto. Here too, examples of EM-based navigation systems may have structural configurations that are different than the navigation system 20 illustrated throughout the drawings.

In other examples, the navigation system 20 and/or the localizer 34 could be based on one or more other types of tracking systems. For example, an ultrasound-based tracking system coupled to the navigation controller 26 could be provided to facilitate acquiring ultrasound images of markers that define trackable features on the tracked objects such that tracked states are communicated to (or interpreted by) the navigation controller 26 based on the ultrasound images. By way of further example, a fluoroscopy-based imaging system (e.g., a C-arm) coupled to the navigation controller 26 could be provided to facilitate acquiring X-ray images of radio-opaque markers that define trackable features such that tracked states are communicated to (or interpreted by) the navigation controller 26 based on the X-ray images.

Furthermore, in some examples, a machine-vision tracking system, including a vision camera can be coupled to the navigation controller 26 and could be provided to facilitate acquiring 2D and/or 3D machine-vision images of structural features that define trackable features such that tracked states are communicated to (or interpreted by) the navigation controller 26 based on the machine-vision images. The machine vision system can be integrated into the camera unit 36, optionally in combination with infrared sensors. The machine vision system can create depth maps and can detect objects with or without trackers. The machine vision system can detect patterns, shapes, colors, computer-codes, tracking geometries, and the like. The machine vision system can detect QR codes, dynamic QR codes, point cloud imagery, and the like.

Various types of tracking and/or imaging systems could define the localizer 34 and/or form a part of the navigation system 20 without departing from the scope of the present disclosure. Furthermore, the navigation system 20 and/or localizer 34 may have other suitable components or structure not specifically recited herein, and the various techniques, methods, and/or components described herein with respect to the optically-based navigation system 20 shown throughout the drawings may be implemented or provided for any of the other examples of the navigation system 20 described herein. For example, the navigation system 20 may utilize solely inertial tracking and/or combinations of different tracking techniques, sensors, and the like. Other configurations are contemplated.

Based on the position and orientation of the trackers 44, 46, 48 and previously loaded data, navigation controller 26 can determine the position of the working end of the surgical tool 22 (e.g., the centroid of a surgical bur) and/or the orientation of the surgical tool 22 relative to the tissue against which the working end is to be applied. In some implementations, the navigation controller 26 forwards these data to a manipulator controller 54. The manipulator controller 54 can then use the data to control the manipulator 12. This control can be like that described in U.S. Patent No. 9,119,655, entitled, "Surgical Manipulator Capable of Controlling a Surgical Instrument in Multiple Modes," or like that described in U.S. Patent No. 8,010,180, entitled, "Haptic Guidance System and Method", the disclosures of which are hereby incorporated by reference.

In one implementation, the manipulator 12 is controlled to stay within a preoperatively defined virtual boundary VB that can be determined by a surgical plan. The virtual boundary VB may be a virtual cutting boundary which defines the material of the target site TS (e.g., the femur F and tibia T) to be removed by the surgical tool 22. More specifically, each of the femur F and tibia T has a target volume of material that is to be removed by the working end of the surgical tool 22. The target volumes are defined by one or more virtual cutting boundaries. The virtual cutting boundaries define the surfaces of the bone that should remain after the procedure. The navigation system 20 tracks and controls the surgical tool 22 to ensure that the working end, e.g., the surgical bur, removes the target volume of material and does not extend beyond the virtual cutting boundary, as disclosed in U.S. Patent No. 9,119,655, entitled, "Surgical Manipulator Capable of Controlling a Surgical Instrument in Multiple Modes," the disclosure of which is hereby incorporated by reference, or as disclosed in U.S. Patent No. 8,010,180, entitled, "Haptic Guidance System and Method", the disclosure of which is hereby incorporated by reference.

The virtual cutting boundary VB may be defined within a virtual model of the anatomy (e.g., the femur F and tibia T), or separately from the virtual model. The virtual cutting boundary may be represented as a mesh surface, constructive solid geometry (CSG), voxels, or using other boundary representation techniques. The surgical tool 22 may be used to cut away material from the femur F and tibia T to receive an implant. The surgical implants may include unicompartmental, bicompartmental, or total knee implants as shown in U.S. Patent No. 9,381,085, entitled, "Prosthetic Implant and Method of Implantation," the disclosure of which is hereby incorporated by reference. Other implants, such as hip implants, shoulder implants, spine implants, and the like are also contemplated. The focus of the description on knee implants is provided as one example. These concepts can be equally applied to other types of surgical procedures, including those performed without placing implants.

The navigation controller 26 can also present on display 28, 29 representations indicating the relative position of the working end of the tool 22 to the tissue. These representations are provided on the clinical application CA to enable the surgical guidance during manipulation of the target site TS.

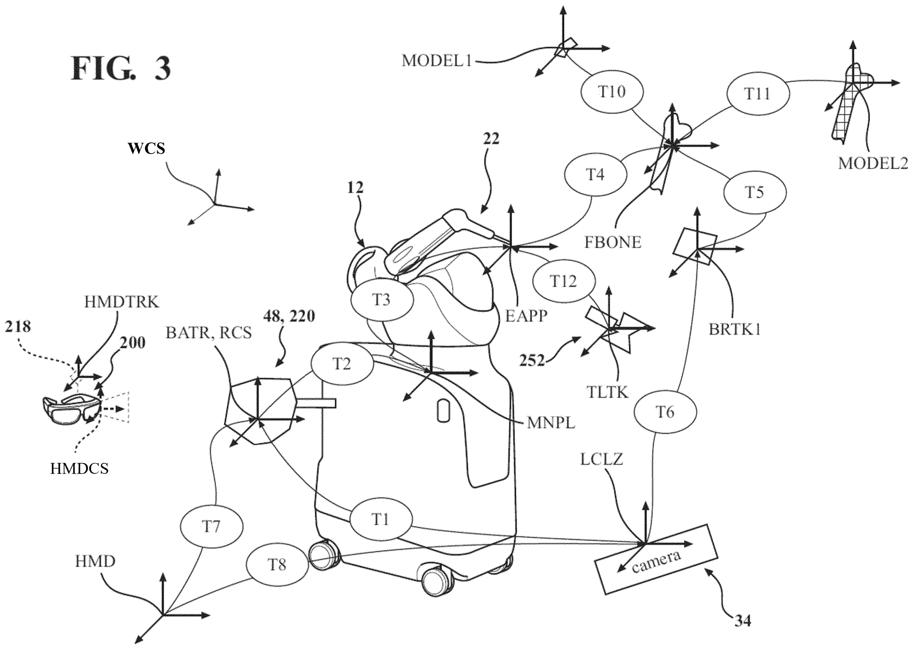

Referring to FIG. 3, tracking of objects can be conducted with reference to a localizer coordinate system LCLZ. The localizer coordinate system has an origin and an orientation (a set of x, y, and z planes). Each tracker 44, 46, 48 and object being tracked also has its own coordinate system separate from the localizer coordinate system LCLZ. Components of the navigation system 20 that have their own coordinate systems are the bone trackers 44, 46 (one of which is shown in FIG. 3) and the base tracker 48. These coordinate systems are represented as, respectively, bone tracker coordinate systems BTRK1, BTRK2 (BTRK1 shown), and base tracker coordinate system BATR. The world coordinate system WCS indicates the coordinate system of the real-world, or room in which the objects are located.

Navigation system 20 monitors the positions of the femur F and tibia T of the patient by monitoring the position of bone trackers 44, 46 rigidly attached to bone. Femur coordinate system is FBONE, and tibia coordinate system is TBONE, which are the coordinate systems of the bones to which the bone trackers 44, 46 are rigidly attached.

Prior to the start of the intraoperative procedure, preoperative images of the femur F and tibia T may be generated (or of other portions of the anatomy in other implementations). The preoperative images can be stored as two-dimensional or three-dimensional patient image data in a computer-readable storage device, such as memory within the navigation system 20. The patient image data may be based on X-ray scans or computed tomography (CT) scans of the patient's anatomy. The patient image data may then be used to generate two-dimensional images or three-dimensional models of the patient's anatomy. The pre-operative data and models may be used for surgical planning purposes and intraoperative guidance. For example, the surgical plan (e.g., tool path or resection volume or boundaries VB), may be planned relative to the virtual model. The virtual model and surgical plan can then be registered to the anatomy using any appropriate registration technique, such as pointer registration, imageless registration, or the like.

In preparation for the intraoperative procedure, the images or three-dimensional models developed from the image data are mapped to the femur coordinate system FBONE and tibia coordinate system TBONE (see transform T11). One of these models is shown in FIG. 3 with model coordinate system MODEL2. These images/models are fixed in the femur coordinate system FBONE and tibia coordinate system TBONE. As an alternative to taking preoperative images, plans for treatment can be developed in the operating room (OR) from kinematic studies, bone tracing, and other methods. The models described herein may be represented by mesh surfaces, constructive solid geometry (CSG), voxels, or using other model constructs.

During an initial phase of the intraoperative procedure, the bone trackers 44, 46 are coupled to the bones of the patient. The pose (position and orientation) of coordinate systems FBONE and TBONE are mapped to coordinate systems BTRK1 and BTRK2, respectively (see transform T5). In one implementation, a pointer instrument 252 (TLTK), such as disclosed in U.S. Patent No. 7,725,162 to Malackowski, et al., hereby incorporated by reference, having its own tracker, may be used to register the femur coordinate system FBONE and tibia coordinate system TBONE to the bone tracker coordinate systems BTRK1 and BTRK2, respectively. Given the fixed relationship between the bones and their bone trackers 44, 46, positions and orientations of the femur F and tibia T in the femur coordinate system FBONE and tibia coordinate system TBONE can be transformed to the bone tracker coordinate systems BTRK1 and BTRK2 so the localizer 34 is able to track the femur F and tibia T by tracking the bone trackers 44, 46. These pose-describing data can be stored in memory integral with both manipulator controller 54 and navigation controller 26.

The working end of the surgical tool 22 has its own coordinate system. In some implementations, the surgical tool 22 comprises a handpiece and an accessory that is removably coupled to the handpiece. The accessory may be referred to as the energy applicator and may comprise a bur, an electrosurgical tip, an ultrasonic tip, a pointer tip, or the like. Thus, the working end of the surgical tool 22 may comprise the energy applicator. The coordinate system of the surgical tool 22 is referenced herein as coordinate system EAPP. The origin of the coordinate system EAPP may represent a centroid of a surgical cutting bur, for example. In other implementations, the accessory may simply comprise a probe or other surgical tool with the origin of the coordinate system EAPP being a tip of the probe. The pose of coordinate system EAPP is registered to the pose of base tracker coordinate system BATR before the procedure begins (see transforms T1, T2, T3). Accordingly, the poses of these coordinate systems EAPP, BATR relative to each other are determined. The pose-describing data can be stored in memory integral with both manipulator controller 54 and navigation controller 26.

Referring to FIG. 2, a localization engine 100 is a software module that can be considered part of the navigation system 20. Components of the localization engine 100 run on navigation controller 26. In some implementations, the localization engine 100 may run on the manipulator controller 54. Localization engine 100 receives as inputs the signals from the localizer 34 and, in some implementations, signals from the tracker controller. Based on these signals, localization engine 100 can determine the pose of the bone tracker coordinate systems BTRK1 and BTRK2 in the localizer coordinate system LCLZ (see transform T6). Based on the same signals received for the base tracker 48, the localization engine 100 determines the pose of the base tracker coordinate system BATR in the localizer coordinate system LCLZ (see transform T1).

The localization engine 100 forwards the signals representative of the poses of trackers 44, 46, 48 to a coordinate transformer 102. Coordinate transformer 102 is a navigation system software module that runs on navigation controller 26. Coordinate transformer 102 references the data that defines the relationship between the preoperative images of the patient and the bone trackers 44, 46. Coordinate transformer 102 can also store the data indicating the pose of the working end of the surgical tool 22 relative to the base tracker 48.

During the procedure, the coordinate transformer 102 receives the data indicating the relative poses of the trackers 44, 46, 48 to the localizer 34. Based on these data, the previously loaded data, and the below-described encoder data from the manipulator 12, the coordinate transformer 102 can generate data indicating the relative positions and orientations of the coordinate system EAPP and the bone coordinate systems, FBONE and TBONE. As a result, coordinate transformer 102 generates data indicating the position and orientation of the working end of the surgical tool 22 relative to the tissue (e.g., bone) against which the working end is applied. Image signals representative of these data are forwarded to displays 28, 29 enabling the surgeon and staff to view this information. In certain implementations, other signals representative of these data can be forwarded to the manipulator controller 54 to guide the manipulator 12 and corresponding movement of the surgical tool 22.

The manipulator 12 has the ability to operate in a manual mode or a semi-autonomous mode in which the surgical tool 22 is moved along a predefined tool path, as described in U.S. Patent No. 9,119,655, entitled, "Surgical Manipulator Capable of Controlling a Surgical Instrument in Multiple Modes," the disclosure of which is hereby incorporated by reference, or the manipulator 12 may be configured to move in the manner described in U.S. Patent No. 8,010,180, entitled, "Haptic Guidance System and Method", the disclosure of which is hereby incorporated by reference.

The manipulator controller 54 can use the position and orientation data of the surgical tool 22 and the patient's anatomy to control the manipulator 12 as described in U.S. Patent No. 9,119,655, entitled, "Surgical Manipulator Capable of Controlling a Surgical Instrument in Multiple Modes," the disclosure of which is hereby incorporated by reference, or to control the manipulator 12 as described in U.S. Patent No. 8,010,180, entitled, "Haptic Guidance System and Method", the disclosure of which is hereby incorporated by reference.

The manipulator controller 54 may have a central processing unit (CPU) and/or other manipulator processors, memory (not shown), and storage (not shown). The manipulator controller 54, also referred to as a manipulator computer, is loaded with software as described below. The manipulator processors could include one or more processors to control operation of the manipulator 12. The processors can be any type of microprocessor or multi-processor system. The term processor is not intended to limit any implementation to a single processor.

A plurality of position sensors S are associated with the plurality of links 58 of the manipulator 12. In one implementation, the position sensors S are encoders. The position sensors S may be any suitable type of encoder, such as rotary encoders. Each position sensor S is associated with a joint actuator, such as a joint motor M. Each position sensor S is a sensor that monitors the angular position of one of six motor driven links 58 of the manipulator 12 with which the position sensor S is associated. Multiple position sensors S may be associated with each joint of the manipulator 12 in some implementations. The manipulator 12 can also include a force/torque sensor coupled between the distal end of the manipulator 12 and the end effector for detecting manual forces/torques exerted on the tool 22 by an operator. The input forces/torques can be used to command movement of the manipulator 12 and/or to detect collisions with the tool 22.

In some modes, the manipulator controller 54 determines the desired location to which the surgical tool 22 should be moved. Based on this determination, and information relating to the current location (e.g., pose) of the surgical tool 22, the manipulator controller 54 determines the extent to which each of the plurality of links 58 needs to be moved in order to reposition the surgical tool 22 from the current location to the desired location. The data regarding where the plurality of links 58 are to be positioned is forwarded to joint motor controllers JMCs that control the joints of the manipulator 12 to move the plurality of links 58 and thereby move the surgical tool 22 from the current location to the desired location. In other modes, the manipulator 12 is capable of being manipulated as described in U.S. Patent No. 8,010,180, entitled, "Haptic Guidance System and Method", the disclosure of which is hereby incorporated by reference, in which case the actuators are controlled by the manipulator controller 54 to provide gravity compensation to prevent the surgical tool 22 from lowering due to gravity and/or to activate in response to a user attempting to place the working end of the surgical tool 22 beyond a virtual boundary VB.

In order to determine the current location of the surgical tool 22, data from the position sensors S is used to determine measured joint angles. The measured joint angles of the joints are forwarded to a forward kinematics module, as known in the art. Based on the measured joint angles and preloaded data, the forward kinematics module determines the pose of the surgical tool 22 in a manipulator coordinate system MNPL (see transform T3 in FIG. 3). The preloaded data are data that define the geometry of the plurality of links 58 and joints. With this encoder-based data, the manipulator controller 54 and/or navigation controller 26 can transform coordinates from the localizer coordinate system LCLZ into the manipulator coordinate system MNPL, vice versa, or can transform coordinates from one coordinate system into any other coordinate system described herein using transformation techniques. In many cases, the coordinates of interest associated with the surgical tool 22 (e.g., the tool center point or TCP), the virtual boundaries, and the tissue being treated, are transformed into a common coordinate system for purposes of relative tracking and display.

In the implementation shown in FIG. 3, transforms T1-T6 are utilized to transform relevant coordinates into the femur coordinate system FBONE so that the position and/or orientation of the surgical tool 22 can be tracked relative to the position and orientation of the femur (e.g., the femur model) and/or the position and orientation of the volume of material to be treated by the surgical tool 22 (e.g., a cut-volume model: see transform T10). The relative positions and/or orientations of these objects can also be represented on the displays 28, 29 to enhance the user's visualization before, during, and/or after surgery.

While the example surgical system 10 has been described with reference to the Figures, the surgical system 10 is not intended to be limited to what is specifically shown and described. For example, the surgical system 10 may not include the manipulator 12 or the navigation system 20 as specifically shown. Other systems are contemplated without departing from the scope of the disclosure.

### II. Head Mounted Device

Referring back to FIGS. 1 and 2, one or more head-mounted devices (HMDs) 200 may be incorporated into the surgical system 10. The HMD may be employed to enhance visualization before, during, and/or after surgery. The HMD 200 is an extended reality device, which can include aspects of augmented reality, mixed reality, virtual reality, and the like. The HMD 200 can be used to visualize the same objects previously described as being visualized on the displays 28, 29, and can also be used to visualize other objects, features, instructions, warnings, etc. The HMD 200 can be used to assist with visualization of the volume of material to be cut from the patient, to help visualize the size of implants and/or to place implants for the patient, to assist with registration and calibration of objects being tracked via the navigation system 20, to see instructions and/or warnings, among other uses, as described further below.

The HMD 200 has a display 208 onto which computer-generated content can be displayed onto a real-world view. In the implementation described herein, the HMD 200 provides on the HMD display 208 a computational holographic/superimposed/overlay of computer-generated content over the real-world view. In one example, the real-world view is acquired by a video camera 214 attached to the HMD. The video camera 214 produces a live video stream of the real-world and the computer-generated content may be combined into video stream of the real world. In such instances, the HMD display 208 may include one or more high-resolution displays positioned in front of the user's eyes. The HMD display 208 may be opaque in such scenarios.

In other implementations, the HMD 200 may implement natural see-through techniques whereby the HMD display 208 is implemented as a transparent lens/visor/waveguide provided between the user's eyes and the real-world. The real-world view is acquired naturally by the user's eyes, and the computer-generated content is provided on the transparent lens/visor/waveguide. Such see-through techniques can include a diffractive waveguide, holographic waveguide, polarized waveguide, reflective waveguide, or switchable waveguide.

The HMD 200 includes a support structure 202, which may be head-mountable in the form of an eyeglass or glasses, headwear or headset, or eyewear (such as a digital contact lens or lenses). The HMD 200 may include additional headbands or supports to hold the HMD 200 on the user's head. In other implementations, the HMD 200 may be integrated into a surgical helmet or other structure worn on the user's head, neck, and/or shoulders. Although not shown, it is contemplated that instead of the HMD 200, an extended reality display screen, such as a monitor, tablet, or hand-held display may be used, which can include similar hardware and capabilities as the HMD 200 described.

The HMD 200 can include an HMD controller 210. The HMD controller 210 can include a content generator 206 that generates the computer-generated content (also referred to as virtual images) and that transmits those images to the user through the HMD display 208. The HMD controller 210 controls the transmission of the computer-generated content to the HMD display 208. The HMD controller 210 may be a separate computer, located remotely from the support structure 202 of the HMD 200, or may be integrated into the support structure 202 of the HMD 200. The HMD controller 210 may be a laptop computer, desktop computer, microcontroller, or the like with memory, one or more processors (e.g., multi-core processors), input devices I, output devices (fixed display in addition to HMD 200), storage capability, etc.

The HMD 200 comprises a plurality of tracking sensors 212 that are in communication with the HMD controller 210. In some cases, the tracking sensors 212 are provided to establish a global coordinate system for the HMD 200, also referred to as an HMD coordinate system. The HMD coordinate system is established by these tracking sensors 212, which may comprise camera sensors or other sensor types, in some cases combined with IR depth sensors, to layout the space surrounding the HMD 200, such as using structure-from-motion techniques or the like. The HMD 200 can also comprise a photo/video camera 214 in communication with the HMD controller 210. The camera 214 may be used to obtain photographic images or video with the HMD 200, which can be useful in identifying objects or markers attached to objects, as well as producing the real-world view. The HMD 200 can comprise an inertial measurement unit IMU 216 in communication with the HMD controller 210. The IMU 216 may comprise one or more 3-D accelerometers, 3-D gyroscopes, and the like to assist with determining a position and/or orientation of the HMD 200 (e.g., head movements) in the HMD coordinate system or to assist with tracking relative to other coordinate systems. The HMD 200 could have a speaker to generate a sound or vibrate to provide an indication to the HMD user of a warning or other information of relevance.

The HMD 200 may also comprise a control input sensors 217. In one example, the control input sensors 217 are configured to recognize biomechanical input, e.g., gesture or eye-based commands from the user. When detecting hand-gestures, the control input sensor 217 is able to sense the user's hands, fingers, or other objects for purposes of determining the user's gesture command and controlling the HMD 200, HMD controller 210, navigation controller 26, and/or manipulator controller 54 accordingly. Gesture commands can be used for any type of input used by the system 10. The gesture commands may be detected below the HMD 200 or may be detected by the camera 214 in front of the HMD 200. The control input sensor 217 to detect gestures can include one or more cameras, infrared sensors, motion sensors, or the like. Gesture controls can include any type of hand or finger motion, including but not limited to: pinching, pointing, swiping, circling, grasping, twisting, or the like.

When detecting eye-based commands, the control input sensor 217 is able to sense the user's eye position, motion, dwell time (stare), gaze and the like, for purposes of determining the user's intended command and controlling the HMD 200, HMD controller 210, navigation controller 26, and/or manipulator controller 54 accordingly. The eye-based commands may be detected using an eye-tracker that is positioned to face the user's eyes, e.g., adjacent to the interior facing HMD display 208. Eye-based controls can include any type of eye-command, including but not limited to: selecting an object, moving an object, or the like. In one example, the user can select a computer-generated object displayed by the HMD 200 by staring at the object continuously for a threshold amount of time. The HMD can also control input sensors 217 in the form of a microphone for recording verbal commands. The HMD controller 210 can process the verbal commands and control the HMD display 208 in response.

Any of the described components of the HMD 200 that can sense information or process sensed information (including but not limited to, the HMD controller 210, the video camera 214, tracking sensors 212, IMU 216, and/or control input sensors 217) can be understood as being part of a "sensing system" of the HMD 220. The sensing system is identified by numeral 219 in FIG. 2.

The HMD 200 can be registered to one or more objects used in the operating room, such as the tissue being treated, the surgical tool 22, the manipulator 12, the trackers 44, 46, 48, the localizer 34, and/or the like. In one implementation, as shown in FIG. 3, a local coordinate system HMDCS is associated with the HMD 200 to move with the HMD 200 so that the HMD 200 is fixed in a known position and orientation in the HMD coordinate system. The HMD 200 can utilize the tracking sensors 212 to map the surroundings and establish the HMD coordinate system. The HMD 200 can then utilize the camera 214 to find objects in the HMD coordinate system. In some implementations, the HMD 200 uses the camera 214 to capture video images of markers attached to the objects and then determines the location of the markers in the local coordinate system HMDCS of the HMD 200 using motion tracking techniques and then converts (transforms) those coordinates to the HMD coordinate system.

In another implementation, a separate HMD tracker 218 (see FIGS. 2 and 3), similar to the trackers 44, 46, 48, could be mounted to the HMD 200 (e.g., fixed to the support structure 202). The HMD tracker 218 can have its own HMD tracker coordinate system HMDTRK that is in a known position/orientation relative to the local coordinate system HMDCS of the HMD 200. Alternatively, the tracker coordinate system HMDTRK could be calibrated to the local coordinate system HMDCS using calibration techniques. In this implementation, the local coordinate system HMDCS becomes the HMD coordinate system and the transforms T7 and T8 would instead originate therefrom. The localizer 34 could then be used to track movement of the HMD 200 via the HMD tracker 218 and transformations could then easily be calculated to transform coordinates in the local coordinate system HMDCS to the localizer coordinate system LCLZ, the femur coordinate system FBONE, the manipulator coordinate system MNPL, or other coordinate system.

Referring back to FIG. 3, a registration device 220 may be provided with a plurality of registration markers 224 (shown in FIG. 1) to facilitate registering the HMD 200 to the localizer coordinate system LCLZ. The HMD 200 locates the registration markers 224 on the registration device 220 in the HMD coordinate system via the camera 214 thereby allowing the HMD controller 210 to create a transform T7 from the registration coordinate system RCS to the HMD coordinate system. The HMD controller 210 then needs to determine where the localizer coordinate system LCLZ is with respect to the HMD coordinate system so that the HMD controller 210 can generate images having a relationship to objects in the localizer coordinate system LCLZ or other coordinate system. The registration device 220 or any technique for registering and/or calibrating the HMD 200 to another coordinate system can be like that described in US Patent No. 10,499,997, entitled "Systems and Methods for Surgical Navigation", the entire contents of which are hereby incorporated by reference in their entirety.

During use, for example, the localizer 34 and/or the navigation controller 26 can send data on an object (e.g., the cut volume model) to the HMD 200 so that the HMD 200 knows where the object is in the HMD coordinate system and can display an appropriate content in the HMD coordinate system. Any of the transforms T1-T12 can be combined to define or register the HMD coordinate system to any object. Once registration is complete, then the HMD 200 can be used to visualize computer-generated content in desired locations with respect to any objects in the operating room. Although these transforms have been described in detail, it is understood that the HMD 200 can operate without requiring any such transforms. The HMD 200 can display content without registering to the bone, or any part of the surgical system 10.

### III. Interactive Techniques for Co-Registration Between HMD and Navigation System

### A. Introduction

Having introduced the system 10 and HMD 200 above, this section now describes various systems, methods, software, and techniques for co-registration of the HMD 200 and the navigation system 20. Co-registration of the HMD 200 and the navigation system 20 enables the pose of the HMD 200 to be known in the localizer coordinate system LCLZ. In turn, the HMD 200 is configured for real-time surgical navigation whereby the HMD 200 can present extended reality virtual objects that can be registered to surgical objects such as the target site TS and/or surgical tools 22. Such virtual objects include but are not limited to: virtual surgical plans, virtual bone models, virtual implant models or plans, medical imaging data, virtual cut guides, tool paths, trajectory guides, virtual depth guides, virtual boundaries, anatomical landmarks, instructions, warnings, notifications, and the like.

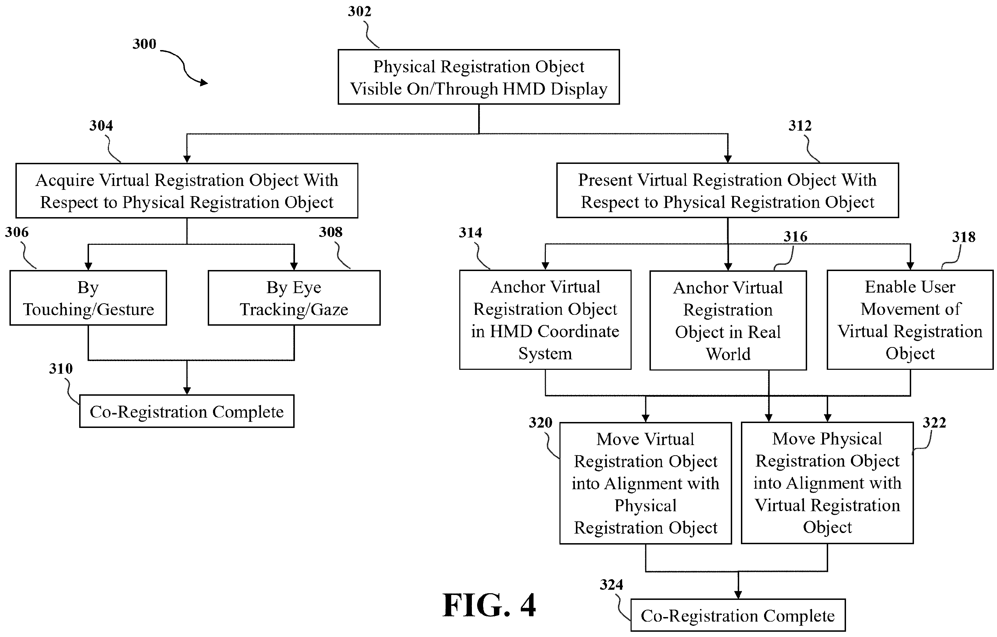

With reference to FIGS. 4-13, these techniques involve determining a spatial relationship between a physical registration object (e.g., a surgical tool) and a virtual registration object. The physical registration object is observed on, or through, the display 208 of the HMD 200. In one implementation, biomechanical control input from the HMD user is sensed to command acquisition of the virtual registration object relative to the physical registration object. In another implementation, the virtual registration object is presented on the HMD display and the physical and virtual registration objects PRO, VRO are brought into alignment, and optionally using a virtual guide object that dynamically guides the user to perform the alignment. The above list is not intended to limit the scope of the claimed subject matter nor identify key features or essential features of the claimed subject matter.

As described herein, the physical registration object PRO is any physical (real) surgical object, or portion thereof, in the operating room that has a state that is known to the navigation system 20. Hence, the state of the physical registration object PRO is known in the localizer coordinate system LCLZ. The physical registration object PRO can be any of the objects described above, including but not limited to any of the tools 22, the robotic manipulator 12, any of the trackers 44, 46, 48, the anatomy or target site TS, a second HMD that is tracked by the navigation system 20, or the camera unit 36 of the navigation system 20. The physical registration object PRO can be a component, feature, part, or portion of any object described, and need not comprise the entirety of the object. The physical registration object PRO can be tracked by the localizer 34 using any of the tracking modalities described above such that its pose is known in the localizer coordinate system LCLZ. In another example, the physical registration object PRO may not be tracked, but may have a known (pre-determined) relationship to the localizer coordinate system LCLZ. For example, the camera unit 36 of the navigation system 20 is not tracked but its state is known in the localizer coordinate system LCLZ. The physical registration object PRO is utilized as an intermediary to facilitate co-registration between the HMD 200 and the navigation system 20. The physical registration object PRO may be, but ideally need not be, a specialized device/tool that is designed for co-registration of the HMD 200 the navigation system 20. Therefore, the techniques described herein advantageously leverage existing tools/objects used in the procedure and contemplate eliminating the need for specialized registration fixtures that can add cost and complexity to the system.

To facilitate the co-registration process, the HMD 200 is configured to enable the physical registration object PRO to be observed on, or through, the display 208. If the HMD 200 comprises the camera 214 that captures a video of the real-world view including the physical registration object PRO, the HMD 200 can present the video on the display 208 that the physical registration object PRO can be observed on the display 208. If the display 208 is a transparent or semi-transparent, the real-world view including physical registration object PRO can be visible by the eyes of the user based on light passing directly through the display 208.

By seeing the physical registration object PRO, the user will be able to take certain actions with the HMD 200 with respect to the pose of the physical registration object PRO to facilitate co-registration. Namely, the user of the HMD 200 is configured to capture and/or utilize one or more virtual registration object(s) VRO that create a spatial relationship with respect to the physical registration object PRO to facilitate co-registration. Based on this spatial relationship, one or more controllers (e.g., the HMD controller 210, the navigation controller 26, or any controller of an intermediary device) can compute spatial transforms between the HMD 200, the virtual registration object VRO, the physical registration object PRO, and the navigation system 20.

As will be described below, the virtual registration object(s) VRO can be acquired relative to the physical registration object PRO and/or presented relative to a real-world view of the physical registration object PRO. Whether presented (predetermined) or acquired (non-predetermined), the state of the virtual registration object(s) VRO will eventually be known in the HMD coordinate system. In either scenario, parameters (e.g., pose, dimension, etc.) of the virtual registration object(s) VRO can be detected and/or tracked by the HMD 200 using the HMD tracking sensors 212 and image analysis or depth sensing, for example.

The method 300 in FIG. 4 provides an overview of various implementations described herein for facilitating co-registration between the HMD 200 and the navigation system 20 utilizing interaction between the virtual registration object(s) VRO and the physical registration object PRO. These steps will be further understood with respect to the various examples described in subsequent sections.

At 302, the physical registration object PRO is made visible on, or through, the HMD display 208. At 304, one technique is utilized wherein the HMD user acquires the virtual registration object(s) VRO with respect to the physical registration object PRO. In one example, the user utilizes biomechanical inputs from the HMD 200. For example, at 306 the user can acquire the virtual registration object(s) VRO by touching or using a gesture on physical registration object PRO. Additionally, or alternatively, at 308, the user can use eye tracking input from the HMD 200, such as gaze, to stare at features or points on the physical registration object PRO. Once the spatial relationship is determined between the virtual registration object(s) VRO and the physical registration object PRO, the controller(s) can facilitate co-registration, at 310.

At 312, another technique may be utilized wherein the virtual registration object(s) VRO is presented on the display of the HMD 200. Here, the virtual registration object(s) VRO is predetermined, and co-registration relies on alignment between the virtual registration object(s) VRO and the physical registration object PRO. In one scenario, at 314, the virtual registration object(s) VRO is anchored in the HMD coordinate system. In another scenario, at 316, the virtual registration object(s) VRO is anchored in the real-world coordinate system. In a third scenario, at 318, the HMD user can move the virtual registration object(s) VRO (in any coordinate system), e.g., using biomechanical control inputs. If the virtual registration object(s) VRO is anchored in the real-world coordinate system at 316, then the physical registration object PRO can be brought into alignment with the virtual registration object(s) VRO, at 322. If the virtual registration object(s) VRO is anchored in the HMD coordinate system (314) or moveable in the HMD coordinate system (318), then either the physical registration object PRO can be brought into alignment with the virtual registration object(s) VRO, the virtual registration object(s) VRO can be brought into alignment with the physical registration object PRO, or a combination of these actions can occur simultaneously. Once the spatial relationship is determined between the virtual registration object(s) VRO and the physical registration object PRO as a result of this alignment, the controller(s) can facilitate co-registration, at 324. The techniques of acquiring the virtual registration object(s) VRO (304) can be combined with the techniques of presenting and aligning the virtual registration object(s) VRO (312). These techniques are not mutually exclusive. During the co-registration process, the presentation methods for the virtual registration object(s) VRO at 314, 316, 318 can be selectively chosen and changed by the HMD user, depending on their preference. For example, the HMD 200 can display a menu or icons to enable the HMD user to change the anchoring/movement scheme.

The controller(s) can be configured to require the virtual registration object(s) VRO to be aligned with or acquired relative to the physical registration object PRO for a threshold duration before acknowledging the alignment/acquisition for co-registration purposes. The threshold duration can ensure that alignment/acquisition is intended by the user and can provide the system with data for multiple frames to provide for accurate results. The threshold duration can be, for example, 1 second, 2 seconds, or greater.

The HMD 200 may be trackerless and not directly trackable by the navigation system 20 prior to co-registration with the localizer coordinate system LCLZ. In this case, prior to co-registration, a pose the HMD 200 is unknown in the localizer coordinate system LCLZ. After co-registration, the pose the HMD 200 can be known in the localizer coordinate system LCLZ such that the HMD 200 is trackable by the navigation system 20. When the HMD 200 is trackerless, the controller(s) can determine the registration transform by utilizing the sensing system 219 of the HMD 200, e.g., video camera 214, tracking sensors 212, IMUs 216, and the like.

In other scenarios, it is possible that the HMD 200 can include the HMD tracker 218 that is detectable by the navigation system 20. In this case, the co-registration process can still be performed for other purposes, such as verification of registration or for performing re-registration, e.g., in case of loss of tracking accuracy or drift occurring on the HMD 200. The co-registration can accurately update the spatial relationship between the HMD 200 and the navigation system 20. Hence, any technique described herein in relation to co-registration can be substituted for "verification" or "re-registration" of the spatial relationship between the HMD 200 and the navigation system 20.

The HMD 200 may or may not be configured to track the physical registration object PRO. For example, prior to co-registration between the HMD coordinate system and the localizer coordinate system, the pose of the physical registration object PRO may be unknown in the HMD coordinate system. In other words, the HMD 200 may not be able to detect a pose of the physical registration object PRO, e.g., due to the physical registration object PRO being configured to be tracked by the navigation system 20 and not tracked by the HMD 200. Alternatively, the pose of the physical registration object PRO may be known in the HMD coordinate system. For example, the HMD camera 214 may detect a shape and/or a pose of the physical registration object PRO, e.g., using RGB and depth sensing/machine vision. The detected shape and/or a pose can then be transformed into the HMD coordinate system. In some cases, the HMD 200 can obtain an identity of the physical registration object PRO, e.g., using a machine-readable code provided on the physical registration object PRO or by matching the shape of the physical registration object PRO to a database of known registration objects. In other examples, the biomechanical control input (e.g., eye or hand/finger tracking) can be utilized to determine the pose of the physical registration object PRO in the HMD coordinate system. For example, the sensing system 219 can detect the HMD user staring at and/or touching the physical registration object PRO. This can be done to acquire virtual registration points relative to the physical registration object PRO to perform the transform. The HMD camera 214 can further be used to monitor/track movement of the physical registration object PRO in the HMD coordinate system.

Similarly, after co-registration between the HMD coordinate system and the localizer coordinate system LCLZ, the pose of the physical registration object PRO may be known or unknown in the HMD coordinate system. The desire for the HMD 200 to track the physical registration object PRO may depend on the requirements of the system or surgical workflow, etc. However, in either case, after co-registration, the pose of the physical registration object PRO relative to the HMD coordinate system can be derived by combining the spatial transforms from the localizer 34 to the physical registration object PRO and the localizer 34 to the HMD 200.

### B. Virtual Guide Object(s)

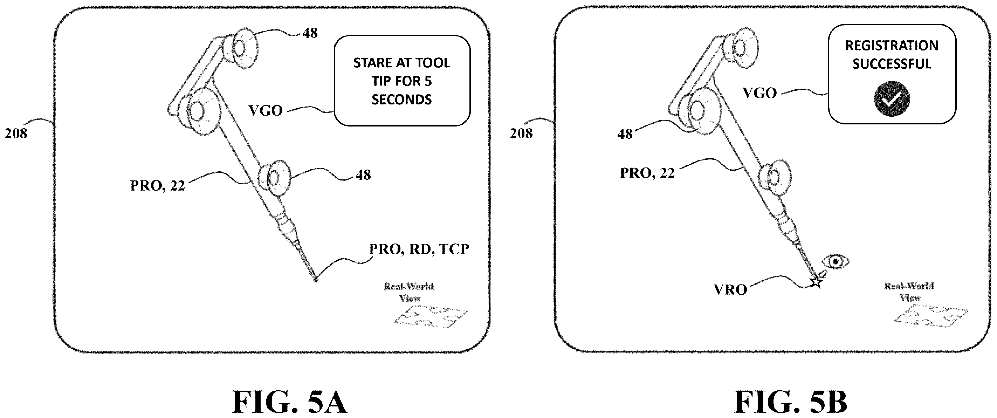

The techniques described herein further provide for virtual guide object(s) VGO to be presented on the display 208 of the HMD 200 to guide the user in commanding acquisition of, or aligning, the virtual registration object(s) VRO with respect to the physical registration object PRO. As shown in FIGS. 5-13, the virtual guide object(s) VGO can be presented as a virtual object on the HMD 200 display relative to the real-world view.

Examples of the virtual guide object(s) VGO can include, but are not limited to, one or more of: a virtual arrow, a virtual path, a virtual animation, a virtual representation of a 3D coordinate system, a virtual compass; and a virtual reticle. For instance, the virtual arrow may be presented to point from the virtual registration object(s) VRO to the physical registration object PRO to guide the user to move the registration objects VRO, PRO into alignment. In another instance, the virtual arrow may be presented to point to a specific feature of the physical registration object PRO to guide the user to acquire the virtual registration object(s) VRO at that point. A virtual compass or coordinate system indicator can provide real-time updated directions in multiple degrees of freedom to facilitate acquisition or alignment of the virtual registration object(s) VRO. A virtual animation can be a series of virtual images (e.g., transparent overlays) that move in the HMD coordinate system to mimic movement of an object, such as either of the registration objects VRO, PRO to guide the user as to the proper movement of either of the registration objects VRO, PRO. The virtual animation can also be presented in a virtual window that is statically located in the HMD coordinate system. A virtual reticle can be like a crosshair or bullseye to indicate where the user should acquire the virtual registration object(s) VRO, for example. In another example, the virtual guide object VGO can be a graphical representation of the physical registration object PRO, such as an image of a tool or a 3D model of a bone, with an indicator to provide where a point should be acquired. Any of these examples can guide the user to manipulate either of the registration objects VRO, PRO in multiple degrees of freedom, up to six degrees of freedom.

In other examples, the virtual guide object(s) VGO can include text, such as messages, instructions, confirmations, errors, warnings, etc., that guide the user during the co-registration process. The text can be presented on the HMD display 208 as an overlay relative to the real-world view. A virtual window can be presented that includes the text. Example text can include "stare at tool tip," "touch tracker marker," "move virtual camera shape into alignment with camera," "move tool 3mm closer", "hold for 2 seconds", "co-registration successful", "inaccurate registration", and the like. These text messages can be provided in addition to, or instead of, the graphical virtual guide object(s) VGO described above.

Whether graphics and/or text, the virtual guide object(s) VGO can be statically presented, or dynamically presented and updated, or a combination thereof. A visual feature or content of the virtual guide object VGO is configured to dynamically change responsive to changes in a spatial relationship between the pose of the physical registration object PRO and the pose of the virtual registration object VRO. If the virtual guide object VGO depends on the location of the physical registration object PRO, the HMD 200 can utilize its sensing system 219 to detect the physical registration object PRO and optionally to determine the spatial relationship between the physical registration object PRO and the virtual registration object VRO.

For graphical or textual guide objects VGO, the visual feature that dynamically changes can be one or more of: a pose of the virtual guide object; a shape of the virtual guide object; a color of the virtual guide object; a direction of the virtual guide object; a magnitude of the virtual guide object; an opacity/transparency of the virtual guide object, and the like. For textual guide objects VGO, the content can be dynamically changed based on sensed conditions. As such, the text can be dynamically changed and updated to reflect: alignment instructions; measurement of displacement between the physical registration object and the virtual registration object; measurement of direction between the physical registration object and the virtual registration object; indication of misalignment; confirmation of alignment, or the like.

The virtual guide object(s) VGO can be presented separately from the virtual registration object VRO and physical registration object PRO or can be presented in a manner that interacts with either registration object VRO, PRO. In one example, the virtual guide object(s) VGO can augment the virtual registration object VRO. For instance, the virtual guide object(s) VGO can be graphically represented as part of the virtual registration object VRO. If the virtual registration object VRO is a virtual outline of a tool 22, the virtual outline can dynamically change color, opacity, or flash/blink depending on the spatial relationship to the physical registration object PRO (tool 22). For instance, if the virtual outline is far from the tool 22, the virtual outline can be colored red. As the virtual outline comes within a threshold distance of the tool 22, the virtual outline can be colored orange. If the virtual outline becomes aligned with the tool 22 such that co-registration is successful, the virtual outline can be colored green. The virtual guide object(s) VGO will be further understood in the examples below.

### C. Biomechanical Input to Capture Virtual Registration Object(s) With Respect to Physical Registration Object

With reference to FIGS. 5-10, described herein are example implementations of co-registration of the HMD 200 and the navigation system 20 by utilizing biomechanical input from the HMD 200 user to acquire the one or more virtual registration object(s) VRO with respect to the physical registration object PRO. In these implementations, the physical registration object PRO is visible to the HMD user on, or through the HMD display 208. However, the virtual registration object(s) VRO are not predetermined. In other words, the virtual registration object(s) VRO are undetermined until the HMD user generates or acquires the virtual registration object(s) VRO. Namely, in these examples, the HMD 200 is configured to sense, with the sensing system 219, the biomechanical control input from the user to command acquisition of the virtual registration object(s) VRO relative to the physical registration object PRO. Based on the relationship between the acquired virtual registration object(s) VRO and the physical registration object PRO, the controller(s) (e.g., the HMD controller 210, the navigation controller 26, or any controller of an intermediary device) are configured to determine a relationship between the HMD coordinate system and the localizer coordinate system LCLZ, or "co-register" the two coordinate systems.

The virtual registration object(s) VRO may be acquired with respect to specific features or points on the physical registration object PRO or with respect to any arbitrary features or points. When acquired, the virtual registration object(s) VRO may be one or more: points, lines, planes, point cloud, area, volume, any complex geometries/shapes, or any combination thereof. For example, to acquire a point, the HMD user can stare at a desired region/point of the physical registration object PRO or touch the desired region/point of the physical registration object PRO. Additionally, the HMD use can stare at many different points or touch various points to form a point cloud. To acquire a line, the HMD user can move their gaze along a line defined by the physical registration object PRO or trace their finger along the line defined by the physical registration object PRO. In another example, the HMD user can acquire a shape by moving their gaze along an outline of the physical registration object PRO or my tracing their finger along the outline of the physical registration object PRO. The user can also utilize a combination of eye tracking and hand tracking techniques to acquire the virtual registration object(s) VRO. For example, the HMD user can simultaneously touch and stare at a point on the physical registration object PRO. The simultaneous input can provide a higher level of accuracy, if needed. In any of these examples, the HMD display 208 can graphically present the virtual registration object(s) VRO after acquisition. The virtual registration object(s) VRO can be overlaid onto the real-world view to indicate to the user that the virtual registration object(s) VRO was acquired and where the virtual registration object(s) VRO was acquired.

In some examples, the virtual registration object(s) VRO are acquired relative to a reference datum RD of the physical registration object PRO. The reference datum RD is any predefined feature of the physical registration object PRO whose pose is known in the localizer coordinate system LCLZ prior to co-registration. In one example, there is data that defines a known spatial relationship between the reference datum RD and a tracker that is coupled directly or indirectly to the physical registration object PRO. The reference datum RD can be, e.g., a tool tip (e.g., of a pointer, drill, bur, router, driver, reamer, impactor, etc.), tracker marker, tool divot or checkpoint, instrument support structure, instrument housing, or the like. However, as described above, the virtual registration object(s) VRO can be acquired relative to any arbitrary feature or points of the physical registration object PRO. Hence, the reference datum RD is not required in every instance.

In one example, as shown in FIGS. 5A and 5B, the physical registration object PRO is a pointer tool 22, or more specifically a tool center point TCP of the pointer tool 22. The TCP serves as a reference datum RD because the distance between the TCP is known relative to the geometry of the tracker 48 coupled to the pointer tool 22. The distance between the TCP and the tracker 48 geometry is known to the navigation system 20. By detecting the tracker 48 geometry, the navigation system 20 can utilize the known spatial relationship between the TCP and the tracker 48 geometry to determine the pose of the TCP. In FIG. 5A, the physical registration object PRO is visible to the HMD user on, or through the HMD display 208. A virtual guide object VGO can be presented to instruct the user to "stare at the tool tip for 5 seconds", for example. In FIG. 5B, the user performs the acquisition of the virtual registration object VRO by staring at the TCP or reference datum RD. In the Figures an icon of an eye is indicated to illustrate that the user is looking at the identified feature. Acquisition of the virtual registration object VRO is indicated in the figures by a star icon. These icons may or may not actually be presented to the HMD user. Once acquisition of the virtual registration object VRO is performed, the virtual guide object VGO is updated to confirm to the user that registration is successful. The HMD 200 is now co-registered to the navigation system 20.

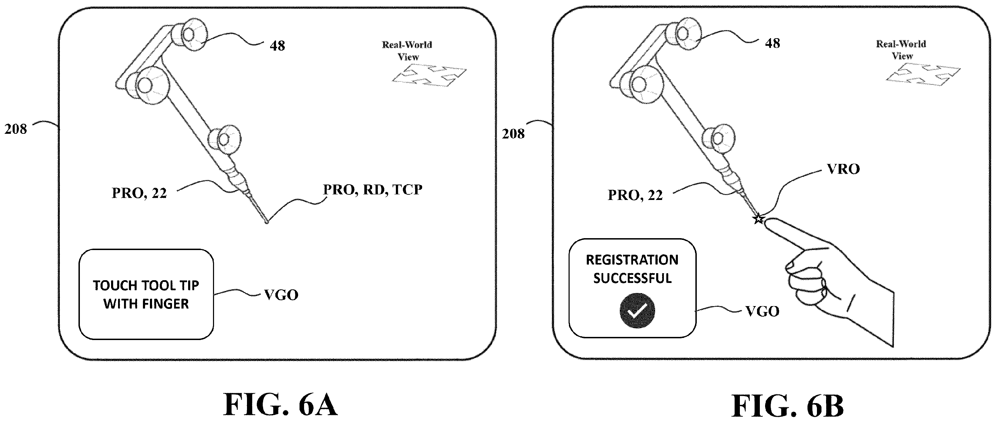

In a similar example, as shown in FIGS. 6A and 6B, the physical registration object PRO is the pointer tool 22, or the tool center point TCP of the pointer tool 22. However, the virtual registration object VRO is acquired using hand-tracking/gesture. In FIG. 6A, a virtual guide object VGO can be presented to instruct the user to "touch tool tip with finger", for example. In FIG. 6B, the user performs the acquisition of the virtual registration object VRO by touching at the TCP or reference datum RD. In FIGS. 6-8, a hand icon is illustrated to indicate that the HMD user is touching the respective portion of the physical registration object PRO. The hand icon may or may not actually be presented to the HMD user. The hand icon can represent the actual hand of the user (seen in the real-world view) or can represent a virtual avatar hand that is derived from gesture. Once acquisition of the virtual registration object VRO is performed, the virtual guide object VGO is updated to confirm to the user that registration is successful. The HMD 200 is now co-registered to the navigation system 20.

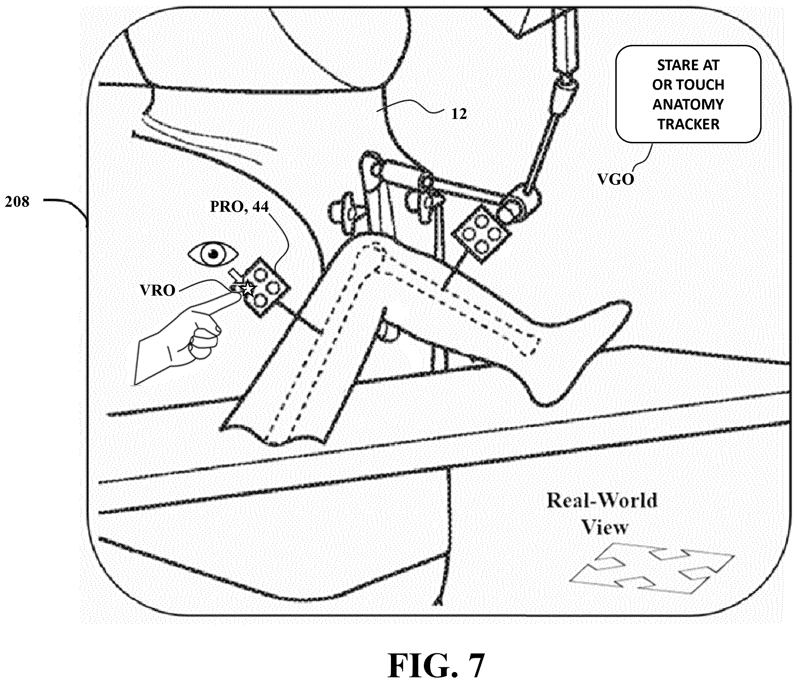

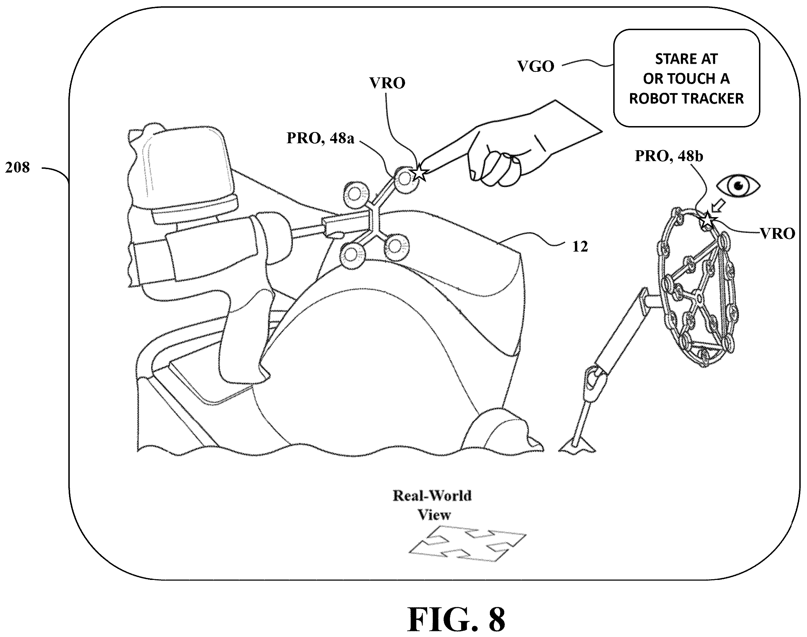

In other examples, as shown in FIGS. 7 and 8, the physical registration object PRO is a tracker that is detectable by the localizer 34, such as any one or more of the anatomy tracker 44, 46, tool tracker 48, or robot tracker 48. The tracker can be coupled to any surgical tool 22, coupled to a base of the robotic manipulator 12, coupled to an end effector of the robotic manipulator 12, coupled to the anatomy TS, coupled to the localizer 34, or a standalone tracker that is not coupled to any object. The virtual registration object(s) VRO can be acquired relative to a feature or any reference datum RD related to the tracker, such as a marker, LED, support structure, tracker arm, tracker coupling mechanism, or tracker mount.

In FIG. 7, the physical registration object PRO is one of the anatomy trackers 44. The virtual registration object VRO is acquired using eye-tracking/gaze and/or hand-tracking/gesture. A virtual guide object VGO can be presented to instruct the user to "stare at or touch anatomy tracker," for example. The user performs the acquisition of the virtual registration object VRO by simultaneously touching and staring at one of the markers of the tracker 44. The controller(s) can determine which marker was subject to acquisition by the virtual registration object VRO using image and/or depth analysis. For example, the controller(s) can compare the depth and pose of the virtual registration object VRO relative to pose of the tracker 44 in the localizer coordinate system LCLZ to find a best-fit match to the markers.

In FIG. 8, the physical registration object PRO is one of the trackers 48a, 48b of the robotic manipulator 12. For example, the tracker 48a can be an end effector tracker coupled to the robotic arm or a base tracker 48b attached to the base of the robotic manipulator 12. Again, the virtual registration object VRO is acquired using eye-tracking/gaze and/or hand-tracking/gesture. A virtual guide object VGO can be presented to instruct the user to "stare at or touch a robot tracker," for example. The user performs the acquisition of the virtual registration object VRO by touching and/or staring at one of the markers of either tracker 48a, 48b. Because there are two different trackers 48a, 48b, the controller(s) can determine which tracker 48a, 48b was subject to acquisition by the virtual registration object VRO using image and/or depth analysis. For example, the controller(s) can compare the depth and pose of the virtual registration object VRO relative to pose of the trackers 48a, 48b in the localizer coordinate system LCLZ to find a best-fit match to the respective tracker and marker.

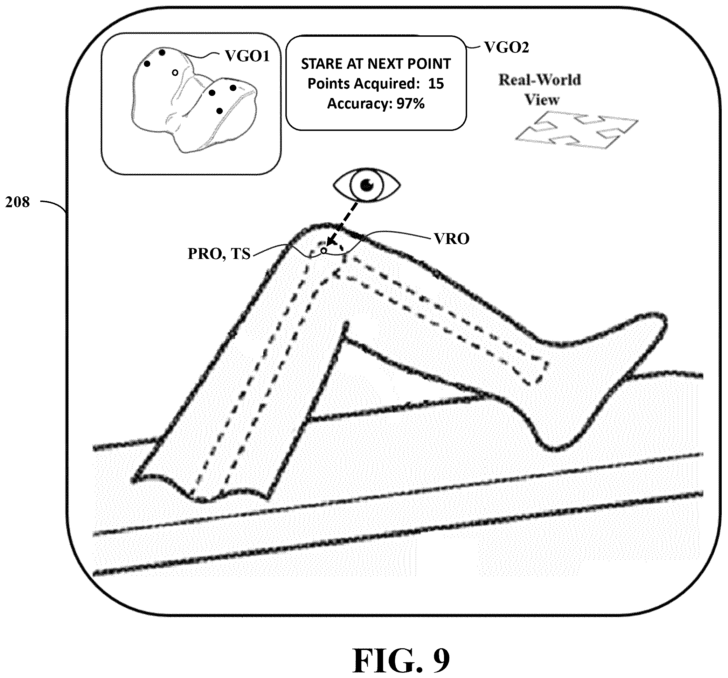

In FIG. 9, the physical registration object PRO is the target site TS of the patient, and more specifically, the bone of a knee joint. The bone is registered to the localizer 34 using any tracking technique described above. In another example, the bone can be registered using machine vision technology whereby the bone surface is imaged and tracked without requiring a tracker attached to the bone. In this example, the virtual registration objects VRO are a series of points that form a point cloud relative to the surface of the bone. As with the previous examples, the virtual registration objects VRO are acquired using eye-tracking/gaze and/or hand-tracking/gesture. A virtual guide object VGO1 can be presented as a virtual panel which shows a 3D model of the bone as well as a sequential display of the various points required for acquisition. The bone model and points can additionally or alternatively be superimposed on the real-world view of the bone. Another virtual guide object VGO2 instructs the user to stare at the respective points (which can be indicated on the bone model) and can indicate how many points have been acquired and/or the accuracy of the point acquisition. The user performs the acquisition of the virtual registration object VRO by staring at or touching the respective feature of the bone denoted by the point indicated by the virtual guide object VGO1. Instead of touching points, the user can move their finger along a tracing path to "paint" any surface. Instead of staring at points, the user can move their gaze along a tracing path to "paint" any surface. The controller(s) can combine the virtual registration objects VRO to form a point cloud or surface mesh that can be registered to the bone. The transform between the HMD 200 and the bone surface can be combined with the transform between the bone surface and the localizer 34 to complete co-registration of the HMD 200 and localizer 34.

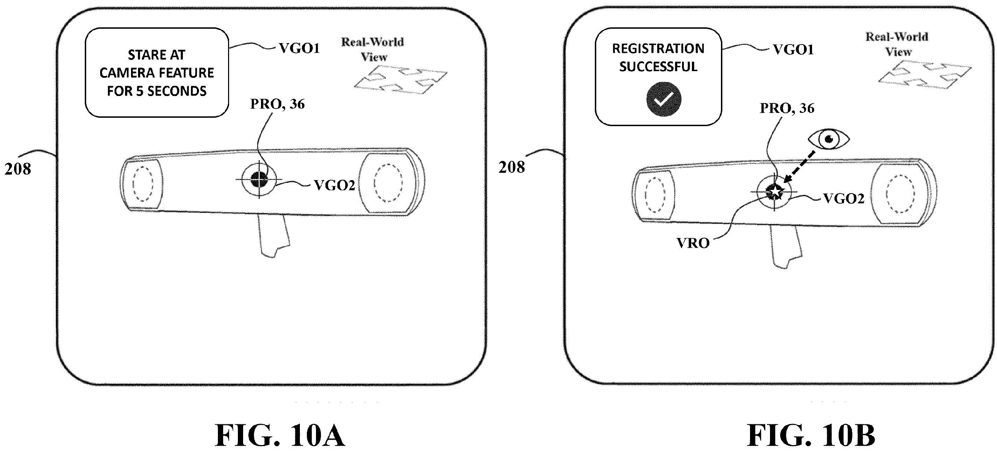

In FIG. 10, the physical registration object PRO is a camera unit 36 of the navigation system 20. Although the camera unit 36 is not an independently tracked device, the pose of the camera unit 36 defines the origin of the localizer coordinate system LCLZ, and hence, the pose of the camera unit 36 is known relative to the localizer coordinate system LCLZ. In this example, the virtual registration object VRO is acquired relative to a feature of the camera unit 36. The feature of the camera unit 36 can be any reference datum RD feature or any arbitrary point. Such features can include a center point of the camera unit 36, a sensor or the camera unit 36, a sticker or indicia on the camera unit 36, or the like. Again, the virtual registration object VRO is acquired using eye-tracking/gaze and/or hand-tracking/gesture. A virtual guide object VGO1 can be presented to instruct the user to "stare at camera feature", for example. Another virtual guide object VGO2 in the form of a reticle can be presented to guide the user as to the location of the acquisition region relative to the camera unit 36. The user performs the acquisition of the virtual registration object VRO by touching and/or staring at the camera unit 36. In another example, the user can move their finger along the housing of the camera unit 36 to acquire the virtual registration object VRO as an outline shape of the camera unit 36. If the virtual registration object VRO correspond to a reference datum RD, the controller(s) compute the transform from the HMD 200 to the reference datum and from the reference datum to the localizer coordinate system LCLZ. If the acquisition point is an arbitrary point, the controller(s) can compare the depth and pose of the virtual registration object VRO relative to pose of the camera unit 36 in the localizer coordinate system LCLZ, the origin of the localizer coordinate system LCLZ, and/or the field of view of the camera unit 36.

Although certain examples have been described and shown regarding biomechanical input to capture virtual registration object(s) with respect to physical registration object, these examples are not limiting. There are numerous other examples of physical registration objects and manners to acquire virtual registration object(s) VRO. For example, as described above, instead of points, the virtual registration object(s) VRO may be lines, planes, point cloud, area, volume, any complex geometries/shapes, or any combination thereof. Other gestures can be utilized such as using multiple fingers or grasping physical registration objects with the hand. More than one hand can be used. Eye-tracking/gaze and/or hand-tracking/gesture detection can be utilized at the same time or at different times during the same co-registration process. Additionally, in any of these examples, a person other the HMD user can move the physical registration object PRO and/or perform acquisition of the virtual registration object VRO using hand-tracking, so long as the other person's hands are visible to the HMD 200. This may be useful, for example, if the surgeon's hands are occupied for another surgical purpose.

### D. Presentation of Virtual Registration Object(s) With Respect to Physical Registration Object

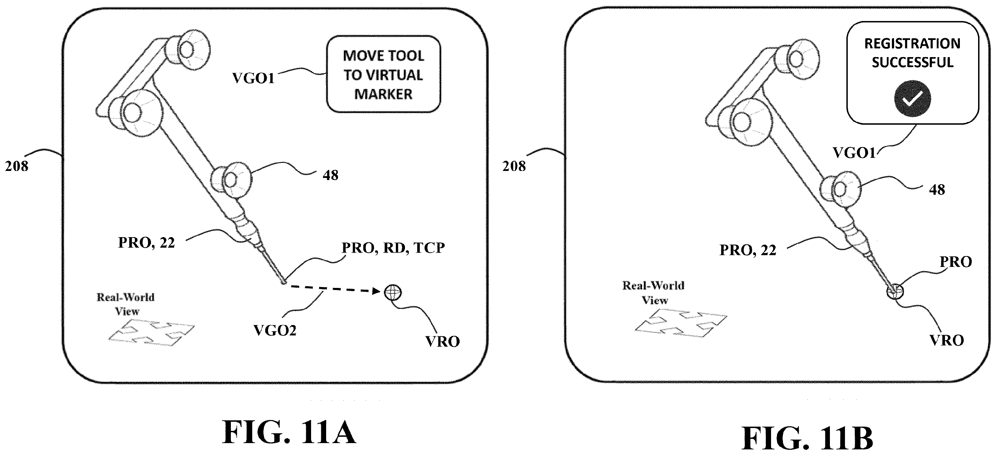

With reference to FIGS. 11-13, described herein are example implementations of co-registration of the HMD 200 and the navigation system 20 by utilizing virtual registration object(s) VRO that are predetermined and configured to be aligned with respect to the physical registration object PRO, or portion thereof. In these implementations, the physical registration object PRO is visible to the HMD user on, or through the HMD display 208. In this implementation, predetermined virtual registration object(s) are presented on the HMD display 208 so that the HMD user can facilitate alignment between the registration objects PRO, VRO. Based on the spatial relationship between the registration objects PRO, VRO pursuant to their alignment, the controller(s) (e.g., the HMD controller 210, the navigation controller 26, or any controller of an intermediary device) are configured to co-register the HMD coordinate system and the localizer coordinate system LCLZ.

As a predetermined object, the virtual registration object(s) VRO may be one or more: points, lines, planes, point cloud, volume, any complex geometries, or any combination thereof. The virtual registration object(s) VRO can be shaped or sized to mimic an entirety or a portion/feature/component of the physical registration object PRO.

The controller(s) will know the pose of the virtual registration object(s) VRO. However, the virtual registration object(s) VRO may be located or manipulated in any of the described coordinate systems. In one example, the virtual registration object(s) VRO may be held static, anchored or spatially locked in the HMD coordinate system (314, FIG. 4). For example, the virtual registration object(s) VRO can remain in the same pose (distance and orientation) relative to the HMD 200, even if the HMD user turns their head or walks in the room. In this implementation, the physical registration object PRO can be brought into alignment with the virtual registration object(s) VRO, the virtual registration object(s) VRO can be brought into alignment with the physical registration object PRO, or a combination of these actions can occur simultaneously.

In another example, the virtual registration object(s) VRO can be anchored relative to the real-world coordinate system (316, FIG. 4). In this example, the virtual registration object(s) VRO is virtually locked in a pose in the real-world such that the HMD user can walk around the virtual registration object(s) VRO and observe it from various poses. For example, the virtual registration object(s) VRO can be virtually anchored over a physical table as though the virtual registration object(s) VRO were a real object located over the table. If the HMD user looks away from the virtual registration object(s) VRO, the virtual registration object(s) VRO will no longer be seen on the HMD display 208. In this example, the physical registration object PRO can be brought into alignment with the anchored virtual registration object(s) VRO while the HMD user observes the registration objects VRO, PRO.

In other instances, the virtual registration object(s) VRO may be dynamically moved by the user (e.g., in the HMD coordinate system or in the real-world coordinate system). The HMD user can utilize the biomechanical control inputs (e.g., gesture or eye-tracking) to move the virtual registration object(s) VRO as desired. The HMD user can rotate, translate, and move the virtual registration object(s) VRO further or closer to their view. This adjustability can enable the HMD user to provide a desired pose for the virtual registration object(s) VRO that can be ergonomically optimized and/or enable the HMD user to move the virtual registration object(s) VRO into alignment with the physical registration object PRO. Additionally, or alternatively, the physical registration object PRO can be brought into alignment with the virtual registration object(s) VRO. In other cases, the HMD user can move their head or change their location in the room to move the virtual registration object(s) VRO into alignment with the physical registration object PRO.

In other implementations, the HMD 200 may be configured to detect the physical registration object PRO. For example, prior to co-registration between the HMD coordinate system and the localizer coordinate system, the HMD 200 may determine the pose of the physical registration object PRO in HMD coordinate system. For example, the HMD camera 214 may detect a shape and/or a pose of the physical registration object PRO, e.g., using RGB and/or depth sensing/machine vision. The detected shape and/or a pose can then be transformed into the HMD coordinate system. In some cases, the HMD 200 can obtain an identity of the physical registration object PRO, e.g., using a machine-readable code provided on the physical registration object PRO or by matching the shape of the physical registration object PRO to a database of known registration objects. In other examples, the biomechanical control input (e.g., eye or hand/finger tracking) can be utilized to determine the pose of the physical registration object PRO in the HMD coordinate system. For example, the sensing system 219 can detect the HMD user staring at and/or touching the physical registration object PRO. This can be done to acquire one or more virtual registration points relative to the physical registration object PRO to perform the transform. The HMD camera 214 can further be used to monitor/track movement of the physical registration object PRO in the HMD coordinate system.

In some situations, the HMD 200 detects the pose of the physical registration object PRO in the HMD coordinate system to perform a coarse/rough registration. The coarse registration may be quickly determined, but less accurate. Thereafter, a fine (more accurate) registration can be performed by virtue of the controller(s) detecting alignment between the pose of the physical registration object and the pose of the virtual registration, according to the techniques described herein. In another example, the HMD 200 detects the pose of the physical registration object PRO in the HMD coordinate system to perform an initial registration. The initial registration may be an accurate registration. Thereafter, a re-registration or registration verification can be performed by virtue of the controller(s) detecting alignment between the pose of the physical registration object and the pose of the virtual registration, according to the techniques described herein. Re-registration may be performed to correct loss of registration, inaccuracies in registration, latency problems, or the like. Verification of registration may be performed to confirm accuracy of the initial registration.

In any of the above examples, alignment between the registration objects VRO, PRO can be detected and/or tracked by the HMD 200 using the HMD tracking sensors 212 and image analysis or depth sensing, for example. As described above, the controller(s) can be configured to require the virtual registration object(s) VRO be aligned with the physical registration object PRO for a threshold duration before acknowledging the alignment for co-registration purposes. Additionally, or alternatively, the controller(s) can require a threshold distance/alignment before acknowledging the alignment for co-registration. For example, the threshold can require that the registration objects VRO, PRO remain within an average 1 mm displacement of each other over a 5 second period. For multi-dimensional virtual registration object(s) VRO, a threshold distance/time can be applied with respect to multiple degrees of freedom.

Figures 11-13 illustrate some examples of how this implementation can be performed. In FIGS 11A and 11B, the physical registration object PRO is the pointer tool 22, or more specifically a tool center point TCP of the pointer tool 22, which also serves as a reference datum RD because the distance between the TCP is known relative to the geometry of the tracker 48 coupled to the pointer tool 22. In FIG. 11A, the pointer tool 22 (i.e., physical registration object PRO) is visible to the HMD user on, or through the HMD display 208. A virtual registration object VRO is presented on the display 208 according to any of the techniques described above (314, 316, 318). Here, the virtual registration object VRO is a single point (or small sphere) that can roughly be the size of the TCP. A virtual guide object VGO1 can be presented to instruct the user to "move the tool to the virtual marker", for example. A second virtual guide object VGO2 can be presented as a virtual arrow that "elastically" extends from the TCP, PRO to the virtual registration object VRO to indicate to the user to move the pointer tool 22 in the indicated direction. As the position and/or orientation of the pointer tool 22 changes, the magnitude and direction of the virtual arrow can correspondingly change. In a different implementation, the virtual guide object VGO can be a static path or "runway" to the virtual registration object VRO. In FIG. 11B, the HMD user (or any other user) moves the pointer tool 22 such that the TCP is aligned with the virtual registration object VRO. The HMD user can hold the pointer tool 22 steady for the threshold duration and/or displacement, if applicable. Once alignment of the virtual registration object VRO is performed, the virtual guide object VGO1 is updated to confirm to the user that registration is successful. The HMD 200 is now co-registered to the navigation system 20.

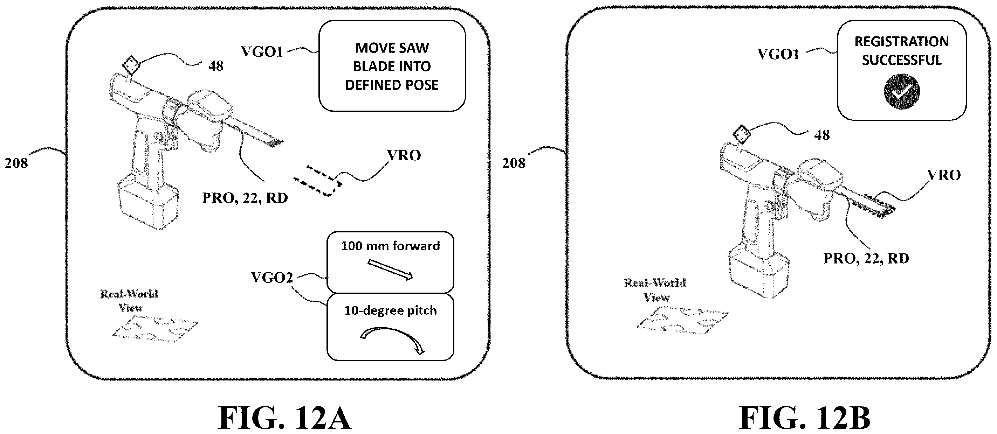

In FIGS 12A and 12B, the physical registration object PRO is the hand-held saw tool 22, or more specifically the saw blade of the saw tool 22, which also serves as a reference datum RD because the distance between the end of the saw blade is known relative to the geometry of the tracker 48 coupled to the saw tool 22. In FIG. 12A, the saw tool 22 (i.e., physical registration object PRO) is visible to the HMD user on, or through the HMD display 208. A virtual registration object VRO is presented on the display 208 according to any of the techniques described above (314, 316, 318). Here, the virtual registration object VRO is a plane that dimensionally mimics the distal portion of the saw blade. Of course, the distal end of the saw blade is just one example of how to define the registration objects PRO, VRO. Other examples are contemplated, such as the entire saw tool 22 for example. A virtual guide object VGO1 can be presented to instruct the user to "move the saw blade into defined pose", for example. A second set of virtual guide objects VGO2 can be presented to provide specific dimensional instructions on how to move the saw tool 22 relative to the virtual registration object VRO. For example, one of the virtual guide objects VGO2 can graphically represent an axial direction and displacement to indicate how far to translate the saw tool 22 and in what direction to translate the tool. Another of the virtual guide objects VGO2 can graphically represent a rotational direction and displacement to indicate how far to rotate the saw tool 22 and in what direction to rotate the tool. As the position and/or orientation of the saw tool 22 changes, the virtual guide objects VGO2 indicators correspondingly change in direction and displacement. The virtual guide object VGO can be implemented in any other way or combination of ways described. In FIG. 11B, the HMD user (or any other user) moves the saw tool 22 such that the saw blade is aligned with the virtual registration object VRO. The HMD user can hold the saw blade steady for the threshold duration and/or displacement, if applicable. Once alignment of the virtual registration object VRO is performed, the virtual guide object VGO1 is updated to confirm to the user that registration is successful. The HMD 200 is now co-registered to the navigation system 20.

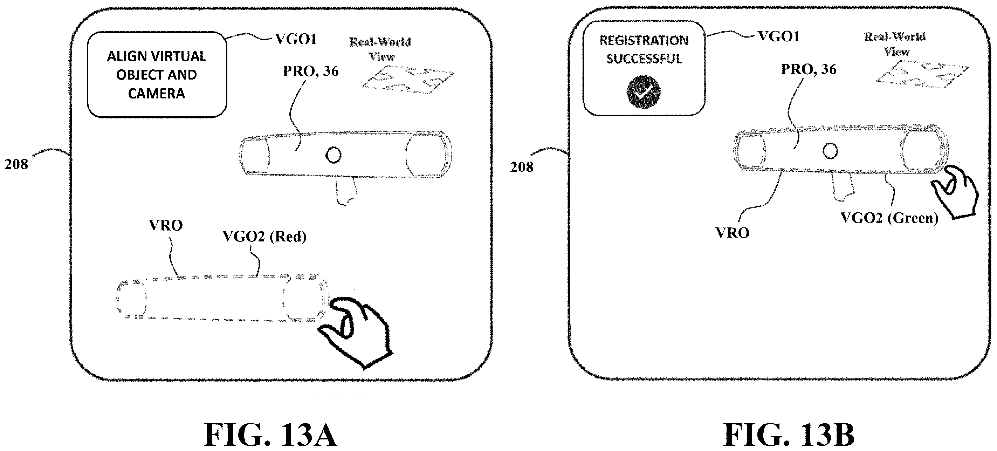

In another example, as shown in FIGS. 13A and 13B, the virtual registration object VRO is moved into alignment with the physical registration object PRO. Here, the physical registration object PRO is the camera unit 35, or more specifically the outer perimeter of the front face of the camera unit 36. In FIG. 13A, the camera unit 36 (i.e., physical registration object PRO) is visible to the HMD user on, or through the HMD display 208. In this example, the camera unit 36 is assumed to be stationary in the room. A virtual registration object VRO is presented on the display 208 in a manner that enables the virtual registration object VRO to be dynamically moved (i.e., 318, FIG. 4). Here, the virtual registration object VRO is an object that dimensionally mimics the outer perimeter of the front face of the camera unit 36. Again, the outer perimeter is just one example of how to define the registration objects PRO, VRO. Other examples are contemplated, such as the shape of one of the sensors of the camera unit 36, for instance. A virtual guide object VGO1 can be presented to instruct the user to "align virtual object to camera", for example. A second virtual guide object VGO2 can be incorporated with and augment the virtual registration object VRO by providing color-coding scheme to indicate the alignment condition. In FIG. 13A, for example, the virtual registration object VRO exhibits a first color (e.g., red) to indicate to the HMD user that the virtual registration object VRO is far from being aligned with the physical registration object PRO. As the position and/or orientation of the virtual registration object VRO approaches the physical registration object PRO, the color coding of the virtual guide object VGO2 will change. Such color coding can be based on threshold displacement calculations performed by the controller(s). Biomechanical control input(s) (eye-tracking or hand-tracking) are utilized to move the virtual registration object VRO towards the physical registration object PRO. In these figures, a hand icon is illustrated to indicate that the HMD user is using gestures to manipulate the pose of the virtual registration object VRO. Again, the hand icon may or may not actually be presented to the HMD user. The hand icon can represent the actual hand of the user (seen in the real-world view) or can represent a virtual avatar hand that is derived from gestures. In FIG. 13B, the HMD user moves the virtual registration object VRO with biomechanical input(s) such that the virtual outer perimeter is aligned with the outer perimeter of the camera unit 36. Once placed, the virtual registration object VRO can selectively be anchored at the placed pose in the real-world coordinate system such that the placed pose is not susceptible to movements of the HMD 200. Once alignment of the virtual registration object VRO is performed, the virtual guide object VGO1 can be updated to confirm to the user that registration is successful. The second virtual guide object VGO2 changes color (e.g., green) to indicate an accurate alignment. The HMD 200 is now co-registered to the navigation system 20.

Although certain examples have been described and shown regarding presentation of predetermined virtual registration object(s) for alignment with respect to the physical registration object, these examples are not limiting. There are numerous other examples of how to define and/or align the registration objects PRO, VRO. For example, the virtual registration object(s) VRO shape or configuration can be defined by the HMD user on-the-fly. For instance, biomechanical control input(s) may be used to coarsely define the shape of the tool 22 and the virtual registration object(s) VRO can immediately be defined and presented as a result. In any of the examples described, biomechanical control input(s) can be used to supplement co-registration via alignment. For example, once alignment occurs, the controller(s) can detect that the user is staring at the aligned registration objects VRO, PRO. This simultaneous or sequential input can provide a higher level of accuracy, if needed. Additionally, in any of these examples, a person other the HMD user can move the physical registration object PRO and/or the virtual registration object VRO using hand-tracking, so long as the other person's hands are visible to the HMD 200. This may be useful, for example, if the surgeon's hands are occupied for another surgical purpose.

The co-registration techniques described in the various sections above take full advantage of specialized XR features for detecting bio-mechanical input, such as gesture or gaze of the headset user. Advantageously, the techniques streamline the co-registration process by reducing steps and eliminating specialized registration hardware, such as specialized co-registration trackers. The bio-mechanical input is a natural, non-disruptive, input to the HMD user and makes the co-registration process less cumbersome than manually acquiring points with a physical pointer while observing the pointer through the HMD display. The various anchoring implementations can provide user flexibility in deciding what the most optimal or ergonomic technique is for co-registration. Additionally, the virtual guides provide a dynamic solution to aid the user in performing the co-registration.

Several implementations have been discussed in the foregoing description. However, the implementations discussed herein are not intended to be exhaustive or limit the invention to any particular form. The terminology which has been used is intended to be in the nature of words of description rather than of limitation. Many modifications and variations are possible in light of the above teachings, and the invention may be practiced otherwise than as specifically described.

The present disclosure also comprises the following clauses, with specific features laid out in dependent clauses, that may specifically be implemented as described in greater detail with reference to the configurations and drawings above.

### CLAUSES

C1. A surgical system, comprising: a navigation system comprising a localizer including a localizer coordinate system, wherein the localizer is configured to track a physical registration object such that a pose of the physical registration object is known in the localizer coordinate system; a head-mounted device (HMD) comprising an HMD coordinate system, a display positionable in front of the eyes of a user of the HMD, and wherein the HMD is configured to: enable the physical registration object to be observed on, or through, the display; detect the physical registration object and obtain a pose of the physical registration object in HMD coordinate system; present a virtual registration object on the display, wherein a pose of the virtual registration object is known in the HMD coordinate system; and one or more controllers coupled to the navigation system and/or the HMD and being configured to: detect alignment between the pose of the physical registration object and the pose of the virtual registration object; and determine a relationship between the HMD coordinate system and the localizer coordinate system in response to detection of alignment between the pose of the physical registration object and the pose of the virtual registration object.
C2.The surgical system of clause C1, wherein: the HMD comprises a sensing system configured to sense a biomechanical control input from the user; and the HMD detects the physical registration object and obtains the pose of the physical registration object in HMD coordinate system by being configured to: utilize the sensing system to sense the biomechanical control input from the user; and command acquisition of one or more virtual points relative to the physical registration object responsive to the sensed biomechanical control input.
C3. The surgical system of clause C2, wherein: the biomechanical control input comprises a gaze of the user; and the HMD commands acquisition of the one or more virtual points relative to the physical registration object by being configured to detect the gaze of the user focused on the physical registration object.
C4. The surgical system of clause C2, wherein: the biomechanical control input comprises a gesture of a hand or finger of the user; and the HMD commands acquisition of the one or more virtual points relative to the physical registration object by being configured to detect the hand or finger of the user physically touching the physical registration object.
C5. The surgical system of any one of clauses C1-C4, wherein: the HMD comprises a camera; and the HMD detects the physical registration object and obtains the pose of the physical registration object in HMD coordinate system by being configured to detect the physical registration object with the camera.
C6. The surgical system of any one of clauses C1-C5, wherein: the HMD obtains the pose of the physical registration object in HMD coordinate system to perform a coarse registration; and the one or more controllers detect alignment between the pose of the physical registration object and the pose of the virtual registration object to perform a fine registration.
C7. The surgical system of any one of clauses C1-C6, wherein: the HMD obtains the pose of the physical registration object in HMD coordinate system to perform an initial registration; and the one or more controllers detect alignment between the pose of the physical registration object and the pose of the virtual registration object to perform a re-registration or verification of the initial registration.
C8. The surgical system of any one of clauses C1-C7, wherein the virtual registration object is a point, an axis, a plane, a volume, or a point cloud.
C9.The surgical system of any one of clauses C1-C8, wherein the virtual registration object is shaped to mimic at least a portion of the physical registration object.
C10. The surgical system of any one of clauses C1-C9, wherein the virtual registration object is virtually anchored in a real-world coordinate system, and the physical registration object is brought into alignment with the virtual registration object.
C11. The surgical system of any one of clauses C1-C10, wherein the virtual registration object is virtually anchored in the HMD coordinate system and: the physical registration object is brought into alignment with the virtual registration object; and/or the virtual registration object is brought into alignment with the physical registration object.
C12. The surgical system of any one of clauses C1-C11, wherein the virtual registration object is configured to be virtually moved in the HMD coordinate system to bring the virtual registration object into alignment with the physical registration object.
C13. The surgical system of any one of clauses C1-C12, wherein: the physical registration object has a reference datum, wherein a pose of the reference datum is known in the localizer coordinate system; and
   the one or more controllers are configured to determine the relationship between the HMD coordinate system and the localizer coordinate system in response to detection of alignment between the pose of the reference datum of the physical registration object and the pose of the virtual registration object.
C14.The surgical system of any one of clauses C1-C13, wherein the one or more controllers are configured to determine the relationship between the HMD coordinate system and the localizer coordinate system in response to detection of alignment for a threshold amount of time.
C15.The surgical system of any one of clauses C1-C14, wherein the physical registration object is a surgical tool that is detectable by the localizer.
C16.The surgical system of clause C15, wherein the surgical tool is separated and disconnected from a patient anatomy.
C17.The surgical system of clause C15, wherein the one or more controllers are configured to determine the relationship between the HMD coordinate system and the localizer coordinate system in response to detection of alignment between a pose of a feature of the surgical tool and the pose of the virtual registration object.
C18. The surgical system of clause C17, wherein: the feature of the surgical tool comprises a tool tip; the virtual registration object is a virtual point; and the one or more controllers are configured to determine the relationship between the HMD coordinate system and the localizer coordinate system in response to detection of alignment between the tool tip and the virtual point.
C19. The surgical system of any one of clauses C1-C18, wherein: the physical registration object is a tracker that is detectable by the localizer; and the one or more controllers are configured to determine the relationship between HMD coordinate system and the localizer coordinate system in response to detection of alignment between a pose of a feature of the tracker and the pose of the virtual registration object.
C20. The surgical system of clause C19, wherein the tracker is: coupled to a surgical tool; coupled to a base of a robotic manipulator; coupled to an end effector of a robotic manipulator; coupled to an anatomy; coupled to the localizer; or a standalone tracker that is not coupled to any object.
C21. The surgical system of any one of clauses C1-C20, wherein; the physical registration object is a camera unit; and the one or more controllers are configured to determine the relationship between the HMD coordinate system and the localizer coordinate system in response to detection of alignment between a pose of a feature of the camera unit and the pose of the virtual registration object.
C22. The surgical system of any one of clauses C1-C21, wherein: the physical registration object is an anatomy; and the one or more controllers are configured to determine the relationship between the HMD coordinate system and the localizer coordinate system in response to detection of alignment between a pose of a feature of the anatomy and the pose of the virtual registration object.
C23.The surgical system of clause C22, wherein the virtual registration object comprises a 3D virtual representation of the anatomy.
C24.The surgical system of any one of clauses C1-C23, wherein the HMD is configured to: present a virtual guide object on the display, wherein the virtual guide object is configured to guide the user to align the pose of the physical registration object and the pose of the virtual registration object.
C25.The surgical system of clause C24, wherein the virtual guide object comprises a visual feature that is configured to dynamically change responsive to changes in a spatial relationship between the pose of the physical registration object and the pose of the virtual registration object.
C26. The surgical system of any one of clauses C1-C25, wherein: prior to determining the relationship between the HMD coordinate system and the localizer coordinate system, a pose the HMD is unknown in the localizer coordinate system; and after determining the relationship between the HMD coordinate system and the localizer coordinate system, the pose the HMD is known in the localizer coordinate system.
C27.The surgical system of clause C26, wherein the HMD is trackerless and not directly trackable by the navigation system prior to determining the relationship between the HMD coordinate system and the localizer coordinate system.
C28.The surgical system of any one of clauses C1-C27, wherein the virtual registration object is combined with a real-world view.
C29.The surgical system of clause C28, wherein the HMD comprises a camera configured to capture a video of the real-world view and the HMD is configured to present, on the display, the video of the real-world view combined with the virtual registration object.
C30.The surgical system of clause C28, wherein the display is a transparent or semi-transparent display such that the real-world view is visible by the eyes of the user based on light passing directly through the display, and wherein the display is configured present the virtual registration object superimposed onto the real-world view.
C31. A surgical system, comprising: a navigation system comprising a localizer including a localizer coordinate system, wherein the localizer is configured to track a physical registration object such that a pose of the physical registration object is known in the localizer coordinate system; a head-mounted device (HMD) comprising an HMD coordinate system, a display positionable in front of the eyes of a user of the HMD, and wherein the HMD is configured to: enable the physical registration object to be observed on, or through, the display; present a virtual registration object on the display, wherein a pose of the virtual registration object is known in the HMD coordinate system and wherein the virtual registration object is virtually anchored in a real-world coordinate system; and one or more controllers coupled to the navigation system and/or the HMD and being configured to co-register the HMD coordinate system and the localizer coordinate system in response to detection of alignment between the pose of the physical registration object and the pose of the virtual registration object.
C32. A surgical system, comprising: a navigation system comprising a localizer including a localizer coordinate system, wherein the localizer is configured to track a physical registration object such that a pose of the physical registration object is known in the localizer coordinate system; a head-mounted device (HMD) comprising an HMD coordinate system, a display positionable in front of the eyes of a user of the HMD, and a sensing system configured to sense a gaze of the user, wherein the HMD is configured to: enable the physical registration object to be observed on, or through, the display; and sense, with the sensing system, the gaze of the user to command movement of a virtual registration object into alignment with the physical registration object; and one or more controllers coupled to the navigation system and/or the HMD and being configured to co-register the HMD coordinate system and the localizer coordinate system in response to detection of alignment between the virtual registration object and the physical registration object.
C33. A surgical system, comprising: a navigation system comprising a localizer including a localizer coordinate system, wherein the localizer is configured to track a surgical tool such that a pose of the surgical tool is known in the localizer coordinate system, wherein the surgical tool comprises a tool tip; a head-mounted device (HMD) comprising an HMD coordinate system, a display positionable in front of the eyes of a user of the HMD, and wherein the HMD is configured to: enable the tool tip of the surgical tool to be observed on, or through, the display; present a virtual registration point on the display, wherein a pose of the virtual registration point is known in the HMD coordinate system; and one or more controllers coupled to the navigation system and/or the HMD and being configured to co-register the HMD coordinate system and the localizer coordinate system in response to detection of alignment between the tool tip and the of the virtual registration point.
C34. A surgical system, comprising: a navigation system comprising a localizer including a localizer coordinate system, wherein the localizer is configured to track a physical registration object such that a pose of the physical registration object is known in the localizer coordinate system; a head-mounted device (HMD) comprising an HMD coordinate system, a display positionable in front of the eyes of a user of the HMD, and wherein the HMD is configured to:
   enable the physical registration object to be observed on, or through, the display;
   present a virtual registration object on the display, wherein a pose of the virtual registration object is known in the HMD coordinate system; and present a virtual guide object on the display, wherein the virtual guide object is configured to dynamically guide the user to align a pose of the physical registration object and the pose of the virtual registration object; and one or more controllers coupled to the navigation system and/or the HMD and being configured to co-register the HMD coordinate system and the localizer coordinate system in response to detection of alignment between the pose of the physical registration object and the pose of the virtual registration object.
C35. The surgical system of clause C34, wherein the virtual guide object comprises one or more of: a virtual arrow; a virtual path; a virtual animation; a virtual representation of a 3D coordinate system; a virtual compass; and a virtual reticle.
C36. The surgical system of any one of clauses C34-C35, wherein the virtual guide object comprises a visual feature that is configured to dynamically change responsive to changes in a spatial relationship between the pose of the physical registration object and the pose of the virtual registration object.
C37. The surgical system of clause C36, wherein the visual feature of the virtual guide object that dynamically changes includes one or more of: a shape of the virtual guide object; a size of the virtual guide object; a length of the virtual guide object; a direction of the virtual guide object; and/or a magnitude of the virtual guide object.
C38. The surgical system of clause C36, wherein the visual feature of the virtual guide object that dynamically changes includes one or more of: a pose of the virtual guide object; a color of the virtual guide object; and/or an opacity of the virtual guide object.
C39. The surgical system of any one of clauses C34-C38, wherein the HMD comprises a camera configured to detect the physical registration object and to determine the spatial relationship between the pose of the physical registration object and the pose of the virtual registration object.
C40. The surgical system of any one of clauses C34-C39, wherein the virtual guide object comprises text that is displayed on the HMD display and that is configured to dynamically change responsive to changes in a spatial relationship between the pose of the physical registration object and the pose of the virtual registration object.
C41. The surgical system of clause C40, wherein the text comprises one or more of: alignment instructions; a measurement of displacement between the physical registration object and the virtual registration object; and/or a measurement of direction between the physical registration object and the virtual registration object.
C42. The surgical system of clause C40, wherein the text comprises one or more of: an indication of misalignment; and/or a confirmation of alignment.
C43. The surgical system of any one of clauses C34-C42, wherein the virtual guide object is presented as being part of the virtual registration object.
C44. The surgical system of any one of clauses C34-C43, wherein the virtual guide object augments the virtual registration object.
C45. The surgical system of any one of clauses C34-C44, wherein the virtual guide object is configured to guide the user with respect to multiple degrees of freedom.

## Claims

1. A surgical system (10), comprising:
a navigation system (20) comprising a localizer (34) including a localizer coordinate system (LCLZ), wherein the localizer (34) is configured to track a physical registration object (PRO) such that a pose of the physical registration object (PRO) is known in the localizer coordinate system (LCLZ);
a head-mounted device (HMD)(200) comprising an HMD coordinate system, a display (208) positionable in front of eyes of a user of the HMD (200), and a sensing system (219) configured to sense a biomechanical control input from the user, wherein the HMD (200) is configured to:
enable the physical registration object (PRO) to be observed on, or through, the display (208); and
sense, with the sensing system (219), the biomechanical control input from the user to command acquisition of a virtual registration object (VRO) relative to the physical registration object (PRO); and
one or more controllers (26, 210) coupled to the navigation system (20) and/or the HMD (200) and being configured to utilize the virtual registration object (VRO) to co-register the HMD coordinate system and the localizer coordinate system (LCLZ).

2. The surgical system (10) of any preceding claim, wherein the virtual registration object (VRO) is a point, a line, a plane, a volume, or a point cloud.

3. The surgical system (10) of any preceding claim, wherein:
the physical registration object (PRO) has a reference datum (RD), wherein a pose of the reference datum (RD) is known in the localizer coordinate system (LCLZ); and
the HMD (200) is configured to sense, with the sensing system (219), the biomechanical control input from the user to command acquisition of the virtual registration object (VRO) that corresponds to the reference datum (RD).

4. The surgical system (10) of any preceding claim, wherein the HMD (200) senses the biomechanical control input by the sensing system (219) being configured to detect a gaze of the user focused on the physical registration object (PRO).

5. The surgical system (10) of any preceding claim, wherein the HMD (200) senses the biomechanical control input by the sensing system (219) being configured to detect a hand or finger of the user physically touching the physical registration object (PRO).

6. The surgical system (10) of any preceding claim, wherein the HMD (200) commands acquisition of the virtual registration object (VRO) in response to sensing, with the sensing system (219), the biomechanical control input for a threshold amount of time.

7. The surgical system (10) of any preceding claim, wherein:
the physical registration object (PRO) is a surgical tool (22) that is detectable by the localizer (34); and
the virtual registration object (VRO) is acquired relative to a feature of the surgical tool (22); and
optionally,
the feature of the surgical tool (22) comprises a tool tip; and
the virtual registration object (VRO) is acquired relative to the tool tip.

8. The surgical system (10) of any one of claims 1-6, wherein:
the physical registration object (PRO) is a tracker (44, 46, 48) that is detectable by the localizer (34); and
the virtual registration object (VRO) is acquired relative to a feature of the tracker (44, 46, 48).

9. The surgical system (10) of any one of claims 1-6, wherein;
the localizer (34) comprises a camera unit (36);
the physical registration object (PRO) is the camera unit (36); and
the virtual registration object (VRO) is acquired relative to a feature of the camera unit (36).

10. The surgical system (10) of any one of claims 1-6, wherein:
the physical registration object (PRO) is an anatomy (F, T, TS) that is registered to the localizer coordinate system (LCLZ); and
the virtual registration object (VRO) is acquired relative to a feature of the anatomy (F, T, TS).

11. The surgical system (10) of any preceding claim, wherein:
prior to co-registration between the HMD coordinate system and the localizer coordinate system (LCLZ), the pose of the physical registration object (PRO) is unknown in the HMD coordinate system; and
after co-registration between the HMD coordinate system and the localizer coordinate system (LCLZ), the pose of the physical registration object (PRO) is known in the HMD coordinate system.

12. The surgical system (10) of any one of claims 1-10, wherein:
prior to co-registration between the HMD coordinate system and the localizer coordinate system (LCLZ), a pose the HMD (200) is unknown in the localizer coordinate system (LCLZ); and
after co-registration between the HMD coordinate system and the localizer coordinate system (LCLZ), the pose of the HMD (200) is known in the localizer coordinate system (LCLZ); and
optionally,
wherein the HMD (200) is trackerless and not directly trackable by the navigation system (20) prior to co-registration between the HMD coordinate system and the localizer coordinate system (LCLZ).

13. The surgical system (10) of any preceding claim, wherein:
the virtual registration object (VRO) is acquired relative to a real-world view of the physical registration object (PRO);
the HMD (200) comprises a camera (214) configured to capture a video of the real-world view; and
the HMD (200) is configured to present the video of the real-world view on the display (208).

14. The surgical system (10) of any one of claims 1-12, wherein:
the virtual registration object (VRO) is acquired relative to a real-world view of the physical registration object (PRO); and
the display (208) is a transparent or semi-transparent display such that the real-world view is visible by the eyes of the user based on light passing directly through the display (208).

15. A head-mounted device (HMD)(200) configured for use with a surgical navigation system (20) comprising a localizer (34) including a localizer coordinate system (LCLZ) and a physical registration object (PRO) whose pose is known in the localizer coordinate system (LCLZ), wherein the HMD (200) comprises:
an HMD coordinate system;
a display (208) positionable in front of eyes of a user of the HMD (200),
wherein the display (208) enables the physical registration object (PRO) to be observed on, or through, the display (208);
a sensing system (219) configured to sense a biomechanical control input from the user; and
an HMD controller (210) configured to:
sense, with the sensing system (219), the biomechanical control input from the user to command acquisition of a virtual registration object (VRO) relative to the physical registration object (PRO); and
utilize the virtual registration object (VRO) to co-register the HMD coordinate system and the localizer coordinate system (LCLZ).
